(19) **Europäisches Patentamt**
**European Patent Office**
**Office européen des brevets**

(11) **EP 2 147 031 B1**

(12) **EUROPEAN PATENT SPECIFICATION**

(45) Date of publication and mention
of the grant of the patent:
**14.09.2011 Bulletin 2011/37**

(51) Int Cl.:
**C08F 290/02** (2006.01)     **A61K 9/00** (2006.01)

(21) Application number: **07711990.7**

(86) International application number:
**PCT/EP2007/002538**

(22) Date of filing: **20.03.2007**

(87) International publication number:
**WO 2008/113390 (25.09.2008 Gazette 2008/39)**

(54) **STEREO PHOTO HYDROGEL, A PROCESS OF MAKING SAID STEREO PHOTO HYDROGEL, POLYMERS FOR USE IN MAKING SUCH HYDROGEL AND A PHARMACEUTICAL COMPRISING SAID POLYMERS**

STEREOPHOTOHYDROGEL, VERFAHREN ZUR HERSTELLUNG DES STEREOPHOTOHYDROGELS, POLYMERE ZUR VERWENDUNG BEI DER HERSTELLUNG EINES DERARTIGEN HYDROGELS UND PHARMAZEUTIKUM, DAS DIE POLYMERE ENTHÄLT

STEREO-PHOTO HYDROGEL, PROCEDE DE FABRICATION DE CET HYDROGEL, POLYMERES UTILISES DANS SA FABRICATION ET PRODUIT PHARMACEUTIQUE CONTENANT CES POLYMERES

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**27.01.2010 Bulletin 2010/04**

(73) Proprietor: **University Of Twente**
**7500 AE Enschede (NL)**

(72) Inventors:
• **HIEMSTRA, Christine**
**NL-7411 DA Enschede (NL)**
• **ZHONG, Zhiyuan**
**NL-7558 SZ Hengelo (NL)**
• **FEIJEN, Jan**
**NL-7552 GD Hengelo (NL)**

(74) Representative: **Van Someren, Petronella F. H. M. et al**
**Arnold & Siedsma**
**Sweelinckplein 1**
**2517 GK Den Haag (NL)**

(56) References cited:
**WO-A-00/48576        WO-A-2005/054318**
**WO-A-2006/007402**

• **CHRISTINE HIEMSTRA, ZHIYUAN ZHONG, LIANGBIN LI, PIETER J. DIJKSTRA AND JAN FEIJEN: "In-Situ Formation of Biodegradable Hydrogels by Stereocomplexation of PEG-(PLLA) 8 and PEG-(PDLA)8 Star Block Copolymers" BIOMACROMOLECULES, vol. 7, 10 October 2006 (2006-10-10), pages 2790-2795, XP002461015**
• **JONG DE S J ET AL: "NOVEL SELF-ASSEMBLED HYDROGELS BY STEREOCOMPLEX FORMATION IN AQUEOUS SOLUTION OF ENANTIOMERIC LACTIC OLIGOMERS GRAFTED TO DEXTRAN" MACROMOLECULES, ACS, WASHINGTON, DC, US, vol. 33, no. 10, 2000, pages 3680-3686, XP000915595 ISSN: 0024-9297**
• **DE JONG S J ET AL: "Biodegradable hydrogels based on stereocomplex formation between lactic acid oligomers grafted to dextran" JOURNAL OF CONTROLLED RELEASE, ELSEVIER, AMSTERDAM, NL, vol. 72, no. 1-3, 14 May 2001 (2001-05-14), pages 47-56, XP004246435 ISSN: 0168-3659**
• **DE JONG S J ET AL: "Physically crosslinked dextran hydrogels by stereocomplex formation of lactic acid oligomers: degradation and protein release behavior" JOURNAL OF CONTROLLED RELEASE, ELSEVIER, AMSTERDAM, NL, vol. 71, no. 3, 28 April 2001 (2001-04-28), pages 261-275, XP004234524 ISSN: 0168-3659**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **BOS G W ET AL: "Tissue reactions of in situ formed dextran hydrogels crosslinked by stereocomplex formation after subcutaneous implantation in rats" BIOMATERIALS, ELSEVIER SCIENCE PUBLISHERS BV., BARKING, GB, vol. 26, no. 18, June 2005 (2005-06), pages 3901-3909, XP004697269 ISSN: 0142-9612**

- **CHRSTINE HIEMSTRA, ZHIUAN ZHONG, PIETER J. DIJKSTRA AND JAN FEIJEN: "Stereocomplex Mediated Gelation of PEG-(PLA)2 and PEG-(PLA) 8 Block Copolymers" MACROMOLECULAR SYMPOSIA, vol. 224, April 2005 (2005-04), pages 119-131, XP002461016**

**Description**

The present invention relates to a stereo photo hydrogel, to a process of making stereo photo hydrogels, to polymers for use in making such hydrogels and a pharmaceutical kit comprising such polymers.

## I. Introduction

Hydrogels have been widely used for biomedical applications, such as tissue engineering and drug delivery, due to their favorable characteristics.[1-3] Hydrogels are water-swollen networks of crosslinked hydrophilic polymers. Their high water content renders them highly biocompatible and also leads to minimal adsorption of proteins. The mechanical properties of hydrogels parallel those of soft tissues, making them particularly interesting for tissue engineering. Hydrogels may be formed in situ, thus allowing easy mixing of cells and bioactive molecules, such as proteins, with the polymer solutions prior to gelation.[4-6] Moreover, in situ hydrogel formation enables the preparation of complex shapes and use of minimally invasive surgery. In situ forming hydrogels have been prepared by physical and chemical crosslinking methods. Physically crosslinked hydrogels include those based on hydrophobic interactions and hydrogen bonds between thermosensitive block or graft polymers[7-11], stereocomplexation between poly(L-lactide) (PLLA) and poly(D-lactide) (PDLA) graft[12] and block copolymers[13-15], inclusion complexation using $\alpha$-dextrin polymers[16-20] and ionic interactions between oppositely charged microparticles[21] or peptides[22]. The crosslinking conditions for these gels are generally very mild, thus allowing the entrapment of labile compounds, such as proteins. However, in general they are mechanically weak compared to chemically crosslinked hydrogels and changes in the external environment (e.g. ionic strength, pH, temperature) may give rise to disruption of the network.

Chemically crosslinked hydrogels have been formed in situ by Michael addition between thiols and acrylates or vinyl sulfones[23-29], reaction between aldehydes and dihydrazides[30] or amines[31], reaction between activated esters and amines[32] and redox initiated radical chain polymerization of (meth)acrylates[33-37]. Photopolymerization of (meth) acrylates[5] using UV-light[38-41] or visible light[42-44] has been mostly used for in situ formation of chemically crosslinked hydrogels. Biodegradable hydrogels prepared by photocrosslinking of poly(ethylene glycol)-poly(lactide) (PEG-PLA) diacrylate derivatives were first reported by the group of Hubbell.[42] More recently, this group has prepared degradable hydrogels by the incorporation of plasmin degradable peptide sequences.[39, 43] When modified with cell-adhesive RGD peptide sequences, these hydrogels supported three-dimensional outgrowth of human fibroblasts embedded as a cluster within the hydrogel. Another type of degradable hydrogel was prepared by copolymerization of a hyaluronic acid methacrylate derivative and PEG diacrylate.[44] Fibroblasts adhered and proliferated when cultured on the RGD functionalized hydrogels. The group of Anseth has done much work on degradable hydrogels based on PEG-PLA dimethacrylates.[40] It was shown that by using combinations of PEG and PEG-PLA dimethacrylates and/or by changing the PLA block length, the hydrogel degradation rate, compressive modulus and crosslinking density could be tuned to provide suitable scaffolds for cartilage tissue engineering.[41] The major advantage of photopolymerization is the spatial and temporal control over the polymerization. However, photopolymerization *in vivo* is hampered by the absorption of UV-light by the skin (> 99%). In clinical applications, fast gelation is desired to prevent diffusion of hydrogel precursors or bioactive molecules to the surrounding tissue. Elisseeff et al. have reported on transdermal photopolymerization of a 20 wt% PEG dimethacrylate aqueous solution injected subcutaneously into nude mice by UV-irradiation for 3 min at 2 mW/cm$^2$ incident light intensity.[45] In this study, high molecular weight PEG (100,000) was used as an additive to prevent rapid diffusion of the gel precursors after injection and to increase the mechanical properties of the photopolymerized hydrogel. A drawback is that it is very difficult to excrete high molecular weight PEG by the kidneys.[46] Elisseeff et al. have studied the UV-light attenuation by the skin using swine skin as a model.[47] The incident light intensity of 100 mW/cm$^2$ was attenuated by the skin to ca. 0.05 mW/cm$^2$. After 3 min of UV-irradiation of a 20 wt% PEG dimethacrylate aqueous solution with 0.04 wt% photoinitiator concentration, a conversion of ca. 10% was reached. The remaining unsaturated bonds may cause toxicity problems and incomplete conversion may result in hydrogels with weak mechanical properties.[48] The polymerization rate may be increased by increasing the photoinitiator concentration or the intensity of the incident light. However, due to their toxicity photoinitiators can only be used at low concentrations (ca. 0.01-0.05 wt%)[49] and the intensity of the UV-light is limited to ca. 5-10 mW/cm$^2$ to prevent cell damage. Visible light is less attenuated by the skin, but efficient initiators with less cytotoxicity are required.[49, 50] Another problem of photopolymerization is that fast polymerization is generally accompanied by substantial heat effects.[48] The resulting temperature rise may cause local cell morbidity and tissue necrosis surrounding the implant.

## II. Summary of the invention

The present invention has for its object to provide a hydrogel which is fast in situ forming and is robust. The present invention is based on the insight that by the combined use of stereo complexation and photo polymerization gelation will be fast and photo polymerization will be facilitated. After rapid formation of the stereo complexed hydrogel, subsequent photo polymerization can be carried out at a relatively low polymerization rate to provide gels with the desired mechanical properties.

Furthermore, the local temperature rise by heat generated by the photo polymerization will be limited. In addition, the photo polymerization may take place at lower initiator concentrations and at lower irradiation intensity as compared to gel formation without using preformed stereo complexes.

Under particular circumstances, the stereo photo hydrogel formed will have increased mechanical properties and if desired prolonged degradation times. Finally, the stereo complexation aids in the photo polymerization of the photo cross-linkable component. The stereo photo hydrogels formed will have increased storage moduli and if desired improved degradation times in comparison to photo hydrogels formed by photo polymerization only. Accordingly, the present invention provides stereo photo hydrogels formed by stereo complexed and photo cross-linked polymers, which polymers comprise at least two types of polymers having at least one hydrophilic component, at least one hydrophobic mutually stereo complexing component, and at least one of the types comprises at least one photo cross-linkable component. Accordingly, this stereo photo hydrogel is formed by stereo complexed and photo cross-linked polymers. These polymers comprise at least two types of polymers. The polymers of both types have at least one hydrophilic component required for forming the hydrogel. The polymers further comprise at least one hydrophobic stereo complexing component which will mutually stereo complex thereby forming the stereo hydrogel. At least one of the two types of polymers comprises at least one photo cross-linkable component. Accordingly, after formation of the stereo hydrogel the polymers of at least one of the two types are cross-linked by photo polymerization thereby forming the stereo photo hydrogel.

The two types of polymers comprise at least one mutually photo cross-linkable component. Accordingly, both types of polymers are mutually photo cross-linked such that both types of polymers are covalently bound thereby forming a very robust stereo-photo hydrogel.

Examples of the hydrophilic component for each of the two types of polymers are PEG, dextran, hyaluronic acid, pullulan, chondroitin sulfate, poly (vinyl alcohol), poly(hydroxyethyl methacrylate), poly(aspartic acid), poly (glutamic acid), poly(acrylic acid), and poly((C1-C6)alkyloxazoline), such as poly(methyl- or ethyl-oxazoline). Preferred is PEG having a number average molecular weight of for instance 10.000-50.000, such as 20-30.000.

An example for the hydrophobic stereo complexing component comprises poly (L-lactide) or poly (D-lactide). In general each poly(L-lactide) or poly (D-lactide) could comprise 3-30 lactyl units per poly (L- or D-lactide). More preferably the number of lactyl units per poly (L- or D-lactide) is 10-20, more preferably 12-16. This depends on the hydrophobic character required for the stereo complexing component relative to the hydrophilic character of the hydrophilic component and the character of the photo cross-linkable component.

The photo cross-linkable component may comprise acrylate, methacrylate, acrylamide and fumarate. Preferred as a photo cross-linkable components are acrylate and methacrylate.

The photo cross-linkable component may be cross-linked using visible or ultraviolet irradiation, depending on the use. For the in-vivo formation of the stereo-photo hydrogel it is preferred to use long wavelength ultraviolet irradiation. When using long wavelength ultraviolet irradiation the intensity of the UV irradiation may be relatively low. For reasons, that due to the prior formation of the stereo hydrogel, photo crosslinking may be carried out at lower rates. The UV irradiation may be as low as 0.05-20 mW/cm$^2$ (when there is a tissue barrier (such as intact skin) or from 2-20 mW/cm$^2$ when there is no tissue barrier. When using visible light, the intensity of the visible light is preferred from 30-100 mW/cm$^2$.

The two types of polymers may have the same or mutually different structures. According to one structure the stereo complexing component and the photo cross-linkable component are both directly linked to the hydrophilic component. According to another structure the photo cross-linkable component is linked to the stereo complexing component which in turn is linked to the hydrophilic component.

With both type of structures hydrogels may be formed, in which the constituting polymers have the form of a triblock or the form of a multi arm structure. When having a multi arm structure, the number of arms is preferably between 3-12, more preferably between 8-10 arm structures.

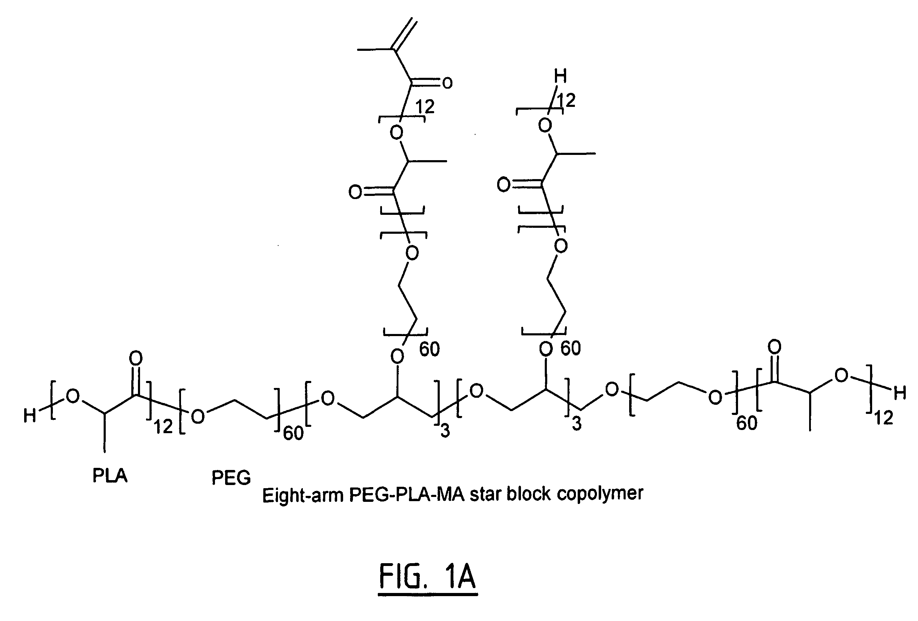

It is noted that various type of structures (of a different composition than the polymers according to the present invention) are disclosed in US 2003/0087985 in figures 1A-1J.

The hydrogels according to the invention have advantageous properties. One of the advantageous properties of the hydrogel is a storage modules G' larger than 1 kPa. Storage moduli G' up to 150kPa may be obtained. Accordingly, the hydrogels may have a storage modules G' within the range of about 1-150kPa, preferably within the range of 1-100kPa.

Another aspect of the present invention relates to the process of making the stereo-photo hydrogels according to the invention. Based on the insight of the present invention the stereo-photo hydrogel is formed by first stereo complexing two types of polymers (as described above). Subsequently, the formed stereo complexed hydrogel is subjected to photo cross-linking thereby forming the stereo-photo hydrogel.

Accordingly, the present invention provides a process of making stereo photo hydrogel comprising the steps of

    a. providing a mixture of at least two types of polymers having at least one hydrophilic component, at least one hydrophobic mutually stereo complexing component and at least one of the types comprises at least one photo cross-linkable component;

b. stereo complexing the two types of polymers, thereby forming a stereo complexed hydrogel; and

c. photo cross-linking the stereo complexed hydrogel using visible or UV irradiation, thereby forming the stereo photo hydrogel.

Accordingly, the two types of polymers are mixed which will, dependent on the circumstances, result in the stereo complexing of both types of polymers within a given time period. In this time period the stereo complexed hydrogel is formed. Subsequently, the stereo complexed hydrogel is subjected to photo cross-linking using irradiation thereby forming the stereo-photo hydrogel. The irradiation may be (low intensity) UV irradiation or visible light.

The two types of polymers both comprise a photo cross-linkable component. Accordingly, both types of polymers are photo cross-linked resulting in a robust stereo photo hydrogel. If desired, the photo cross-linking may be carried out in the presence of a photo initiator. When the photo cross-linking is to take place within the animal or human body it is preferred to use an in situ compatible photo initiator (preferably in an amount of 0.001 to 0.05 wt%). Suitable examples are 2.2-dimethoxy-2-phenylacetophenone (Irgacure 651), 1-hydroxycyclohexyl phenyl ketone (Irgacure 184), 2-methyl-1-[4-(methylthio)  phenyl]-2-(4-morpholinyl)-1-propanone  (Irgacure  907),  and  2-hydroxy-1-(hydroxyethoxy)phenyl]-2methyl-1-propanone (Darocur 2959), camphorquinone (CQ) with ethyl 4-$N,N$-dimethylaminobenzoate (4EDMAB) and triethanolamine (TEA) and the photosensitizer isopropyl thioxanthone.

The mixture of the two types of polymers may comprise the polymers in any suitable concentration for subsequent stereo complexing. In general the polymer concentration is within the range of 5-30wt-v%. Preferably, the polymer concentration is within the range of 10-20wt-v%. Dependent on the type of polymers, their structure and the concentration the stereo complexing time is within the range of 1 minute-3 days. Accordingly, it is possible in relation to the objective use of the photo stereo hydrogels to select the time of stereo complexing as required. Particularly, short or relatively short times for stereo complexing may be selected when it is desired to include within the stereo complexed hydrogel and subsequently within the stereo-photo hydrogel a component which may leach out or may be diffused out of the stereo photo hydrogel over an extended period of time and not during the stage of forming the stereo complex and the formation of the stereo-photo hydrogel. Then relatively short times for stereo complexing may be selected. Preferably, the time for stereo complexing is within 2 hours to 1 day, and more preferably within 4-10 hours.

The stereo-photo hydrogels will be subject to hydrolysis and thereby degradation of the hydrogel. Hydrolysis preferably takes place by hydrolysis of the hydrophobic component and more preferably by hydrolysis of the polylactide chain. When the stereo-photo hydrogel is formed of polymers having structures in which the photo cross-linkable component is at the end of some or all hydrophobic components, then degradation will result in a clear solution within a period of time of about 1 day-7 weeks, such as 1-3 weeks.

If the stereo-photo hydrogel is formed by structures in which the hydrophobic component and the photo cross-linkable component are both directly linked to the hydrophilic component, then degradation by hydrolysis of the hydrophobic component will result in a swollen hydrogel which will subsequently degrade by hydrolysis of the photo cross-linkable component and then form a clear solution. Degradation from the swollen hydrogel into the clear solution will take 2-30 weeks, such as 5-25 weeks, more preferably 7-21 weeks. Clearly, the swollen hydrogel has different properties than the stereo-photo hydrogels.

The hydrogels may comprise a pharmaceutically active agent or a moiety that binds a pharmaceutically active agent. Pharmaceutically active agents may be any pharmaceutically active compound used for therapy, diagnosis or prophylaxis of a human or animal body. The pharmaceutically active agent may comprise cells and biologically active molecules such as proteins, antibodies and the like.

Another aspect of the present invention relates to the polymers of both types as discussed above. Both types of polymers are suitable for use in making a hydrogel which may comprise a pharmaceutical active agent or moiety that binds a pharmaceutically active agent. Such hydrogel may have the form of a medicament for the treatment of the human or animal body. The treatment is related to the biological activity of the pharmaceutically active agent as discussed above.

Finally, the present invention relates to a pharmaceutical kit which may be used for in-situ or in-vitro forming of a stereo-photo hydrogel according to the present invention. Such pharmaceutical kit comprises two containers each comprising one of two types of polymers having at least one hydrophilic component, at least one hydrophobic mutually stereo complexing component, and at least one of the types comprises at least one photo cross-linkable component. When the stereo-photo hydrogel is to comprise the pharmaceutically active agent or a moiety binding a pharmaceutically active agent, then it is preferred that the pharmaceutically active agent is contained in one or both containers for each of the two types of polymers. If possible or needed, the pharmaceutically active agent or the moiety that binds a pharmaceutically active agent is present in a separate container. Prior to the formation of the stereo hydrogel and the stereo-photo hydrogel, the content of one or more containers is mixed.

Mentioned and other features and characteristics of the stereo hydrogel, stereo-photo hydrogel and the process of making these hydrogels will be further illustrated by the following examples which are given for information purposes only and are not intended to restrict to any extend the present invention. In these examples reference will be made to the figures wherein:

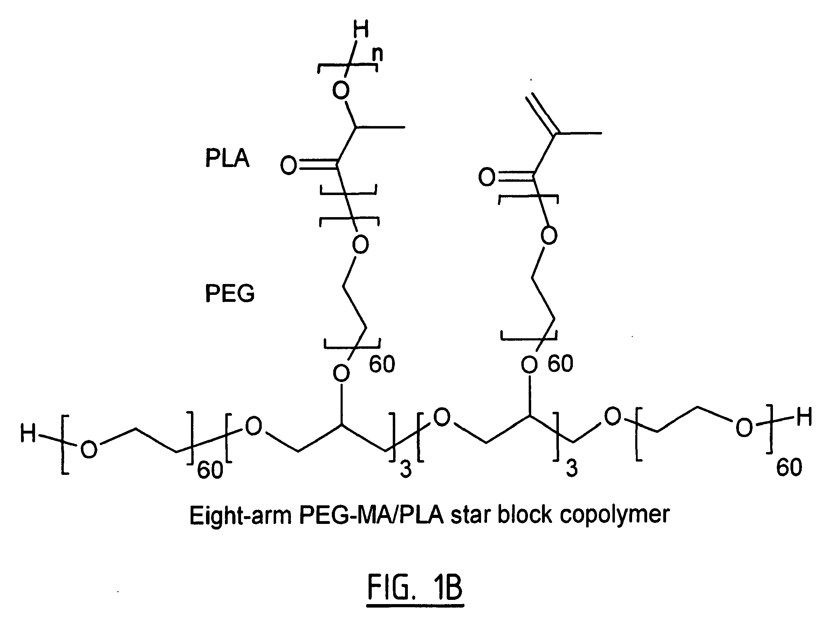

figure 1 shows molecular structures of (A) eight-arm PEG-PLA$_{12}$-MA star block copolymers and (B) PEG-MA/PLA$_n$ (n = 12 or 16) star block copolymers. As an example three methacrylate groups per molecule are drawn;

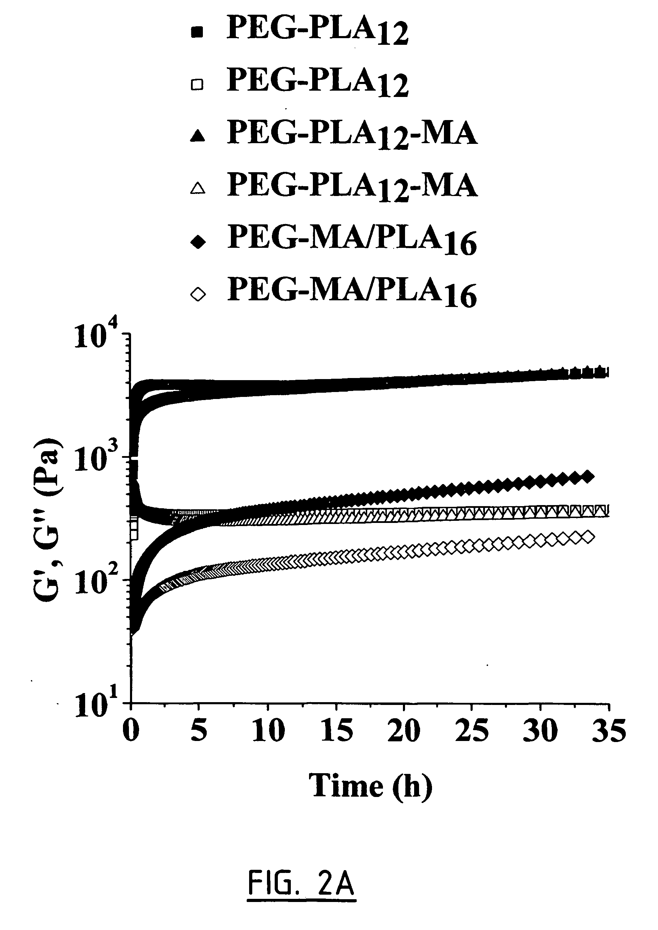

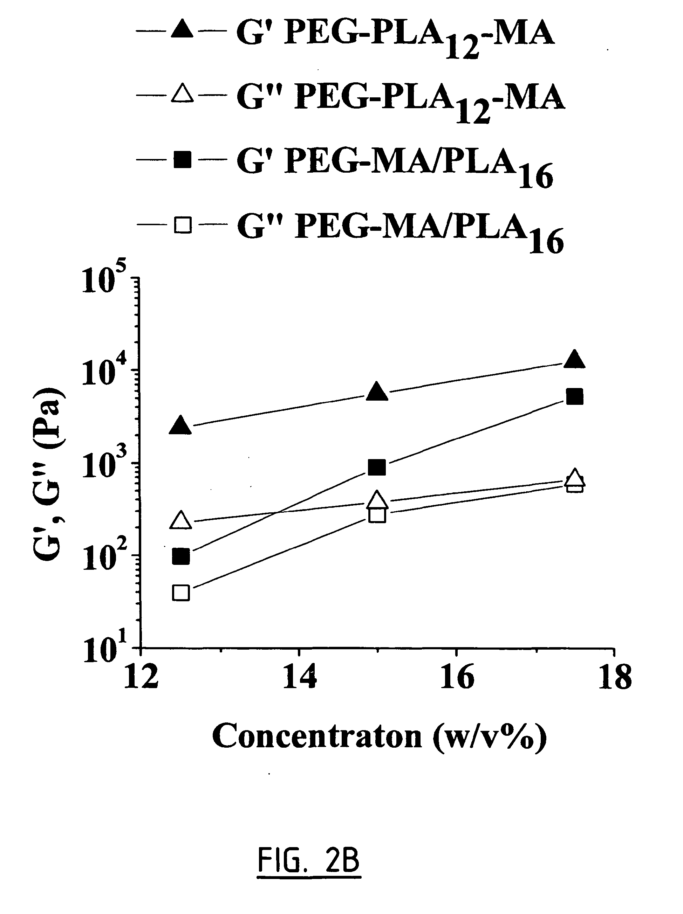

figure 2 shows the storage modulus (G') and loss modulus (G") of stereo hydrogels containing equimolar amounts of PEG-PLLA$_{12}$ and PEG-PDLA$_{12}$, PEG-PLLA$_{12}$-MA and PEG-PDLA$_{12}$-MA, or PEG-MA/PLLA$_{12}$ and PEG-MA/PDLA$_{12}$ star block copolymers in HEPES buffered saline (pH 7) at 37 °C. (a) PEG-PLA$_{12}$, PEG-PLA$_{12}$-MA and PEG-MA/PLA$_{16}$ at 15 w/v% polymer concentration as a function of time; (b) PEG-PLA$_{12}$-MA and PEG-MA/PLA$_{16}$, 48 h after mixing as a function of the polymer concentration;

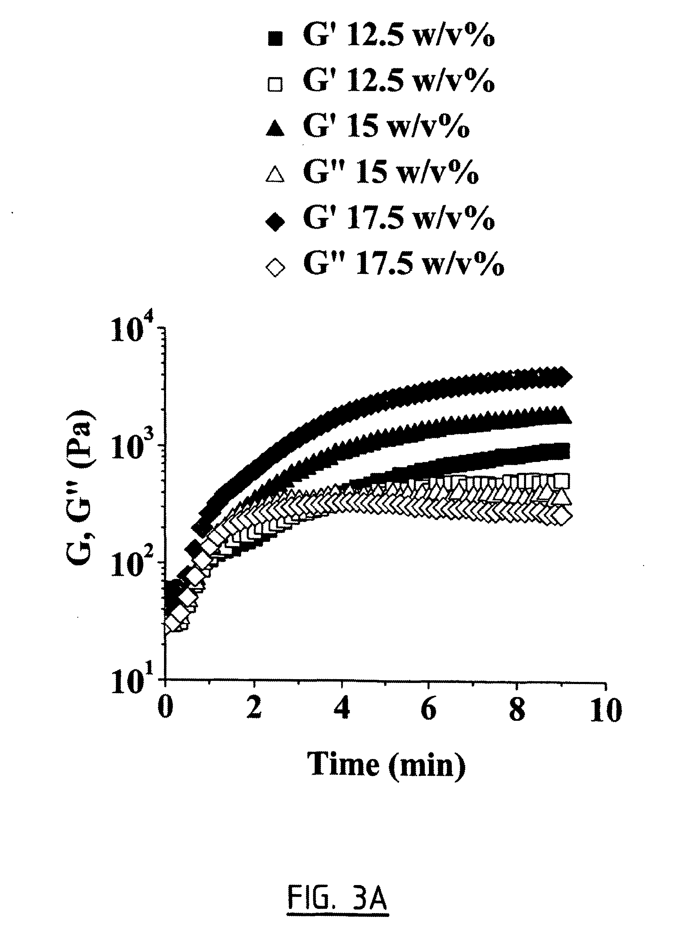

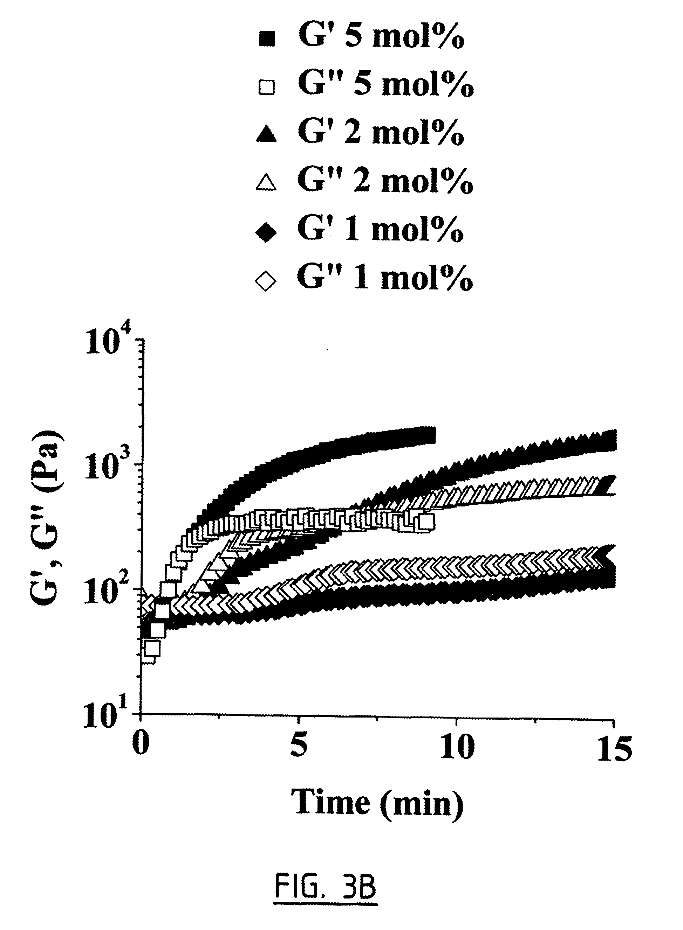

figure 3 shows the storage modulus (G') and loss modulus (G") as a function of UV-irradiation time (350-400 nm, 16 mW/cm$^2$) of PEG-PLLA$_{12}$-MA solutions in HEPES buffered saline (pH 7) at 37°C. (a) 12.5, 15 and 17.5 w/v% polymer concentration and 5 mol% initiator concentration (with respect to the methacrylate groups); (b) 1, 2 and 5 mol% initiator concentration and 15 w/v% polymer concentration;

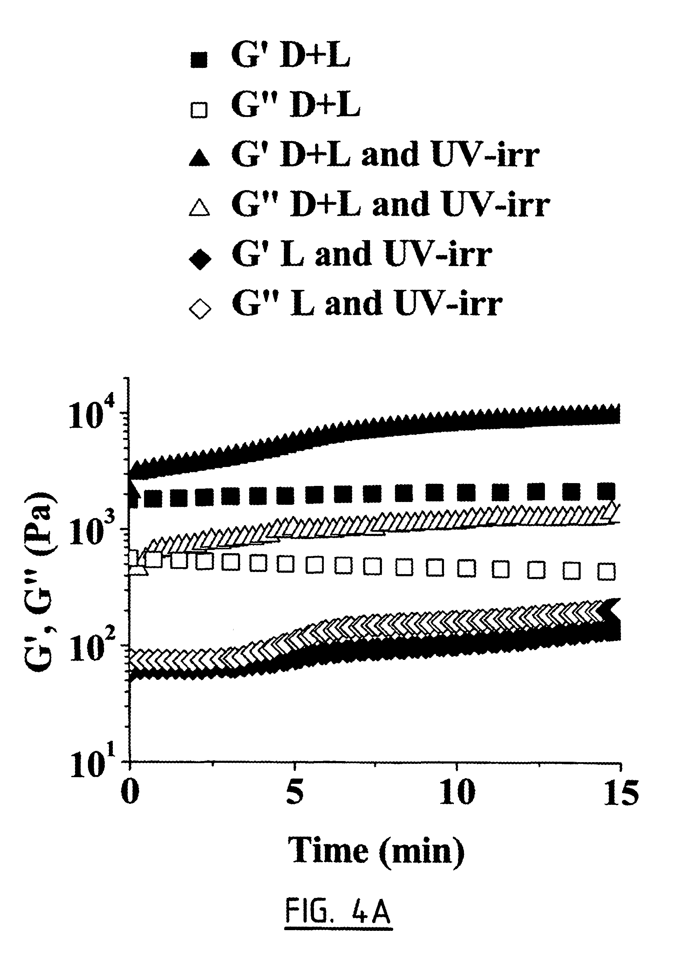

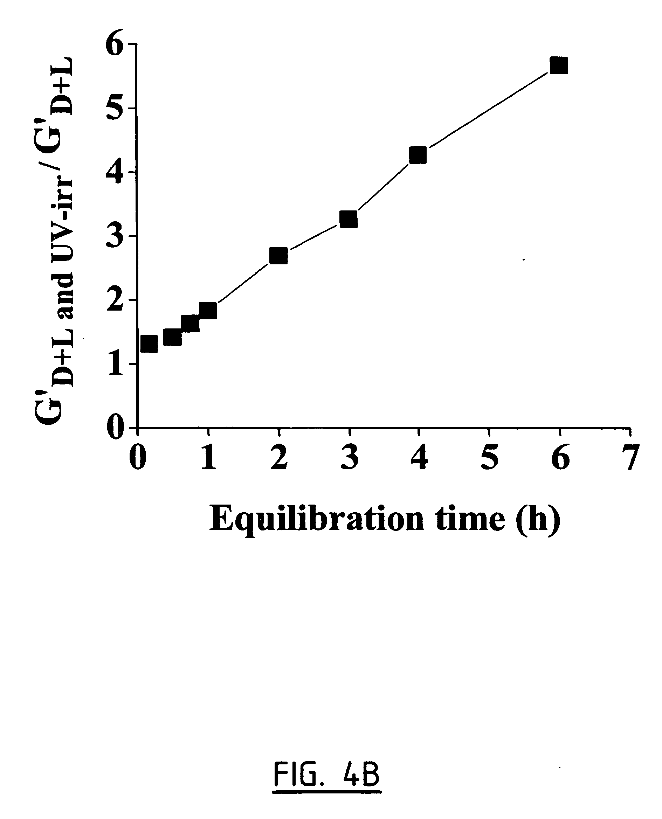

figure 4 shows rheology of UV-irradiated (350-400 nm, 16 mW/cm$^2$) PEG-PLA$_{12}$-MA in HEPES buffered saline (pH 7) at 15 w/v% polymer concentration and 37°C. (a) Storage modulus (G') and loss modulus (G") as a function of time of a stereo hydrogel (D+L) and a stereo-photo hydrogel (D+L and UV-irr) after 10 min of stereocomplex equilibration, and a UV-irradiated PEG-PLLA$_{12}$-MA solution (L) at 1 mol% initiator concentration (with respect to the methacrylate groups); (b) ratio of the storage modulus plateau value of a stereo-photo hydrogel (G'$_{D+L\ and\ UV-irr}$) and the storage moduli plateau value of a stereo hydrogel (G'$_{D+L}$) after 8 min of UV-irradiation as a function of the stereocomplexation equilibration time;

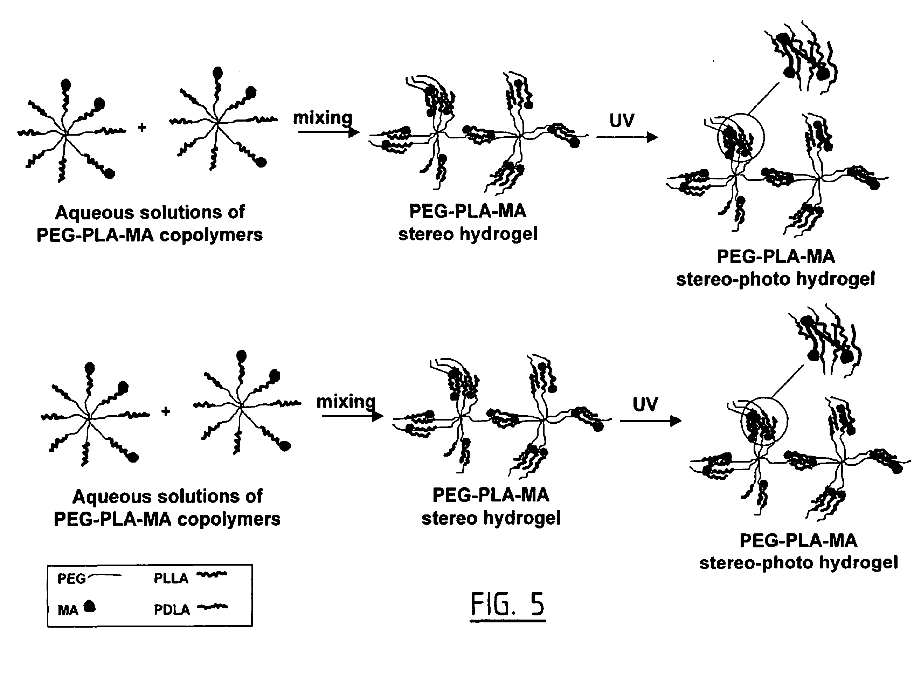

figure 5 shows a schematic representation of the preparation of stereo and stereo-photo hydrogels based on PEG-PLA-MA or PEG-MA/PLA star block copolymers;

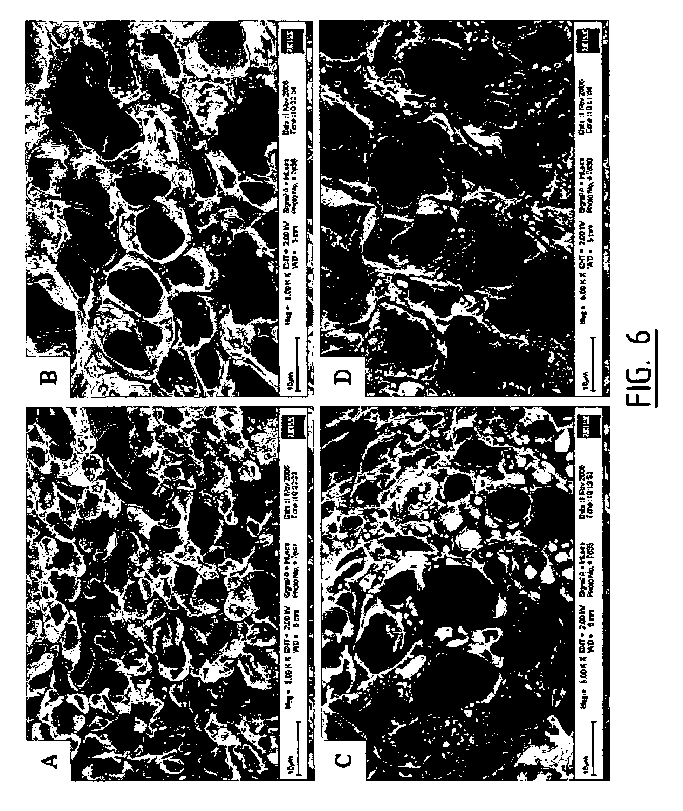

figure 6 shows SEM photos of freeze-dried photo polymerized hydrogels prepared in HEPES buffered saline (pH 7) at 15 w/v% polymer concentration and 8 mol% initiator concentration (with respect to the methacrylate groups) by UVA-irradiation for 10 min (stereo hydrogels were equilibrated for ca. 15 min after mixing of the enantiomeric solutions). (A) PEG-PLA$_{12}$-MA stereo-photo hydrogel; (B) PEG-PLLA$_{12}$-MA photo hydrogel; (C) PEG-MA/PLA$_{16}$ stereo-photo hydrogel; (D) PEG-MA/PLLA$_{16}$ photo hydrogel;

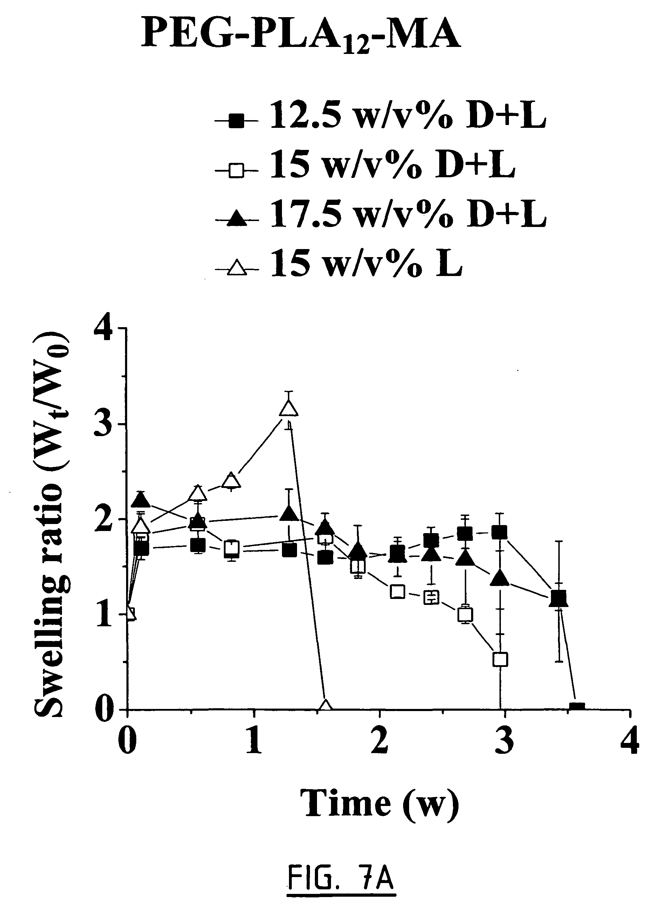

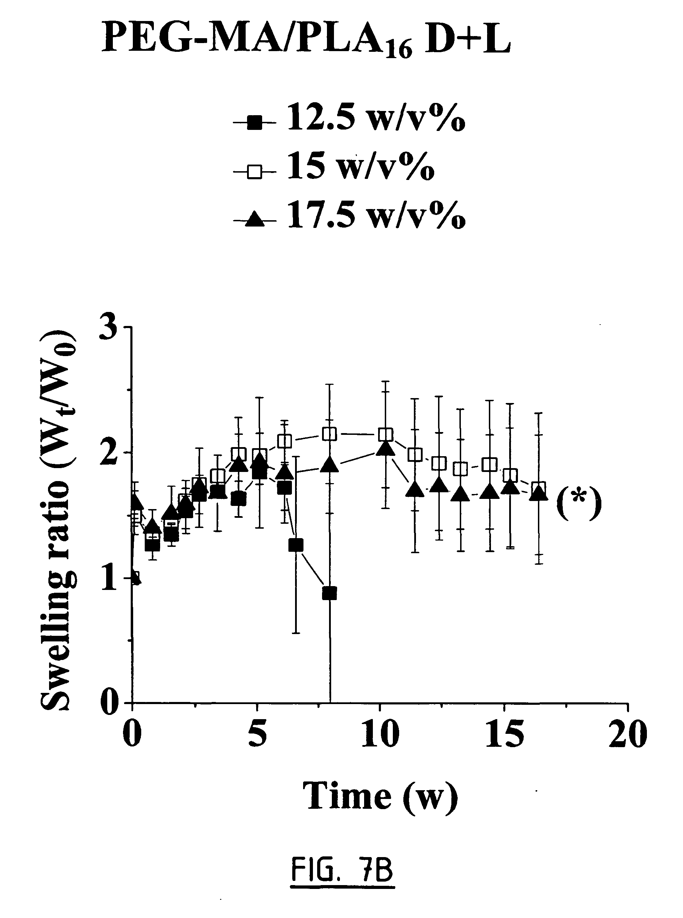

figure 7 shows swelling ratio (W$_t$/W$_0$) profiles of photo polymerized hydrogels prepared in HEPES buffered saline (pH 7) at 8 mol% initiator concentration (with respect to the methacrylate groups) and 37°C by UVA-irradiation for 10 min (stereo hydrogels were equilibrated for ca. 15 min after mixing of the enantiomeric solutions). (a) PEG-PLA$_{12}$-MA stereo-photo hydrogels at 12.5, 15 and 17.5 w/v% polymer concentration and PEG-PLLA$_{12}$-MA photo hydrogels at 15 w/v% polymer concentration; (b) PEG-MA/PLA$_{16}$ stereo-photo hydrogels at 12.5, 15 and 17.5 w/v% polymer concentration. (*) PEG-MA/PLA$_{16}$ stereo-photo hydrogels at 15 and 17.5 w/v% polymer concentration retained their integrity after 16 weeks; and

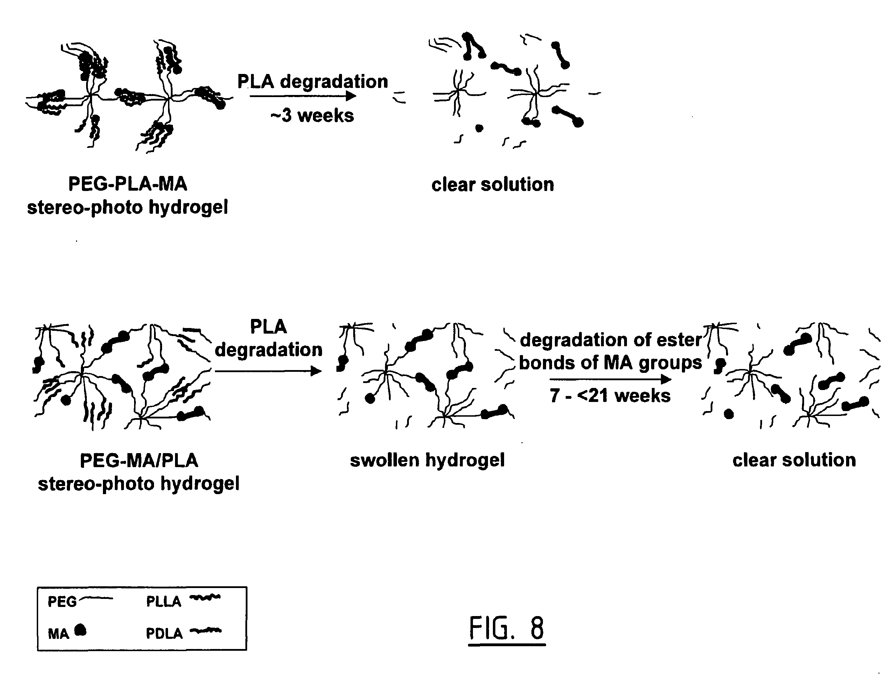

figure 8 shows a schematic representation of the degradation of stereo-photo hydrogels based on PEG-PLA-MA or PEG-MA/PLA star block copolymers.

## III. EXAMPLES

### III.1 Materials

L-lactide and D-lactide were obtained from Purac and recrystallized from dry toluene. Eight-arm star PEG (M$_{n,\ NMR}$ = 21,800) was supplied by Nektar and used as received. The single site Zn-complex catalyst (Zn(Et)[OC$_6$H$_4$(CH$_2$N(Me)$_2$)-2, Me-4]) was kindly provided by Professor G. van Koten of the University of Utrecht (The Netherlands). Methacrylic anhydride was purchased from Merck and Irgacure 2959 from Ciba Specialty Chemicals. Both were used as received. Dichloromethane (DCM) and triethylamine (TEA) were dried over calcium hydride and potassium hydroxide, respectively, and distilled prior to use. Eight-arm poly(ethylene glycol)-poly(L-lactide) and poly(ethylene glycol)-poly(D-lactide) star block copolymers with 12 lactyl units per poly(lactide) (PLA) block (PEG-PLLA$_{12}$ and PEG-PDLA$_{12}$, respectively) were prepared as reported previously (M$_{n,\ PEG}$ = 21,800).[53]

### III.2 Synthesis.

PEG-PLLA$_{12}$-MA and PEG-PDLA$_{12}$-MA were synthesized by partial methacrylation of the hydroxyl groups of PEG-PLLA$_{12}$ and PEG-PDLA$_{12}$, respectively, according to the procedure reported by Lin-Gibson et al.[54] Typically, PEG-PLLA$_{12}$ (5.0 g, 0.174 mmol, dried overnight under vacuum over phosphorous pentoxide) was dissolved in 18 ml of DCM. A solution of TEA (0.171 g, 1.690 mmol) in 1 ml of DCM was added and the reaction mixture was cooled in an ice bath. Subsequently, a solution of methacrylic anhydride (0.244 g, 1.583 mmol) in 2 ml of DCM was added dropwise. The reaction mixture was stirred for 2 days at 30 °C and the product was recovered by precipitation in a mixture of cold diethyl ether/hexane/methanol (10/1/1 v/v). Degree of methacrylation: 40%, yield: 88%. $^1$H NMR (CDCl$_3$): δ 1.4 (m, CH(CH$_3$)OH end group PLA), 1.5 (m, CHCH$_3$), 1.9 (s, C(CH$_3$)=CH$_2$), 3.6 (m, PEG methylene protons), 4.2-4.3 (m, CH$_2$OCO, linking unit PEG-PLA), 4.3-4.4 (q, CH(CH$_3$)OH end group PLA), 5.1 (m, CHCH$_3$), 5.6 and 6.2 (C(CH$_3$)=CH$_2$) PEG-MA/PLLA and PEG-MA/PDLA, in which both MA and PLA blocks are directly linked to PEG, were synthesized by ring opening polymerization of lactide using partially methacrylate functionalized eight-arm star PEG (PEG-MA). For the synthesis of PEG-MA, typically, PEG (16.0 g, 0.734 mmol) was dissolved in 33 ml of DCM. A solution of TEA (0.442 g, 4.368 mmol) in 1 ml of DCM was added and the reaction mixture was cooled in an ice bath. Subsequently, a solution of methacrylic anhydride (0.654 g, 4.242 mmol) in 2 ml of DCM was added dropwise. The reaction mixture was stirred for 2 days at 30°C and the product was recovered by precipitation in a mixture of cold diethyl ether/hexane/methanol (10/1/1 v/v). Degree of methacrylation: 42%, yield: 90%. $^1$H NMR (CDCl$_3$): δ 1.9 (s, C(CH$_3$)=CH$_2$), 3.6 (m, PEG methylene protons), 4.2 (m, CH$_2$OCO, linking unit PEG-MA), 5.6 and 6.2 (C(CH$_3$)=CH$_2$) PEG-MA/PLLA and PEG-MA/PDLA were synthesized by ring opening polymerization of L-lactide and D-lactide, respectively, in DCM at room temperature, initiated by the remaining hydroxyl groups of PEG-MA (dried overnight under vacuum over phosphorous pentoxide). The single site Zn-complex Zn(Et)[OC$_6$H$_3$(CH$_2$Me$_2$)-2-Me-4] was used as a catalyst. Typically, PEG-MA (3.0 g, 0.136 mmol) (degree of methacrylation 42%) and L-lactide (0.532 g. 3.694 mmol) were dissolved in 14 ml of DCM ([LA]$_0$ = 0.25 M). A solution of single site Zn-complex catalyst (0.064 g, 0.247 mmol) was added in 1 ml of DCM and the reaction mixture was stirred for 1 h. The polymerization was terminated by the addition of an excess of glacial acetic acid and the polymer was precipitated in a mixture of cold diethyl ether/methanol (20/1 v/v). Lactide conversion: 95%, yield: 85%. $^1$H NMR (CDCl$_3$): δ 1.4 (m, CH(CH$_3$)OH end group PLA), 1.5 (m, CHCH$_3$), 1.9 (s, C(CH$_3$)=CH$_2$), 3.6 (m, PEG methylene protons), 4.2 (m, CH$_2$OCO, linking unit PEG-MA), 4.2-4.3 (m, CH$_2$OCO, linking unit PEG-PLA), 4.3-4.4 (q, CH(CH$_3$)OH end group PLA), 5.1 (m, CHCH$_3$), 5.6 and 6.2 (C(CH$_3$)=CH$_2$)

Characterization. $^1$H NMR spectra (CDCl$_3$) were recorded on a Varian Inova Spectrometer (Varian, Palo, Alto, USA) operating at 300 MHz. The number of lactyl units per PLA block was calculated based on the methyl protons of lactyl units (δ 1.4-1.5) and the methylene protons of PEG (δ 3.6). The number of methacrylate groups per PEG molecule was determined based on the methylene protons of PEG (δ 3.6) and the methylene protons of the methacrylate group (δ 5.6 and 6.2).

Critical gel concentrations (CGCs) were determined as described before.[53] Briefly, polymer solutions were prepared by dissolving the polymers in deionized water overnight. Subsequently, polymer solutions of equimolar amounts of PEG-PLLA-MA and PEG-PDLA-MA, or PEG-MA/PLLA and PEG-MA/PDLA star block copolymers were mixed and equilibrated overnight. The CGCs were determined at room temperature by inverting the vials. When the sample showed no flow within 20 s, it was regarded as a gel.

Rheology experiments were performed on a US 200 Rheometer (Anton Paar), as described previously.[53] Briefly, a flat plate measuring geometry (25 mm diameter, gap 0.5 mm), a frequency of 1 Hz and a strain of 1% were used. Polymer solutions in HEPES buffered saline (pH 7.0, 100 mM, adjusted to 300 mOsm with NaCl) containing equimolar amounts of PEG-PLLA-MA and PEG-PDLA-MA, or PEG-MA/PLLA and PEG-MA/PDLA star block copolymers were mixed, homogenized, quickly applied to the rheometer and measured at 37°C.

Combined UV-irradiation and rheology experiments were performed on a US 200 Rheometer (Anton Paar) equipped with a UV-light source (350-400 nm, 16 mW/cm$^2$). The samples were irradiated from above. A flat plate measuring geometry (10 mm diameter, gap 0.1 mm), a frequency of 1 Hz and strains of 1% or 5% were used. Both strains are within the linear viscoelastic region. Both PEG-PLA-MA or PEG-MA/PLA stereocomplexed hydrogels (stereo hydrogels) and solutions of PEG-PLLA-MA or PEG-MA/PLLA single enantiomers in HEPES buffered saline were UV-irradiated and at the same time measured at 37°C. Irgacure 2959 was used as photoinitiator at a concentration of 1-5 mol% relative to the methacrylate groups. The stereo hydrogels were measured 10 min after mixing the enantiomeric solutions, unless mentioned otherwise.

Hydrogels for scanning electron microscopy (SEM) experiments and swelling/degradation tests were prepared similarly in a 96 wells plate with sample volumes of 125 μl, resulting in cylinders of ca. 4 mm in height and 6 mm in diameter. PEG-PLA$_{12}$-MA or PEG-PLA$_{16}$/MA stereo-photo hydrogels were prepared by UVA-irradiation (250 mW/cm$^2$) for 10 min of the stereo hydrogels (equilibrated for ca. 15 min after mixing of the enantiomeric solutions) with 8 mol% initiator concentration (with respect to the methacrylate groups) prepared in HEPES buffered saline. Photo hydrogels were formed similarly by UVA-irradiation of PEG-PLLA$_{12}$-MA or PEG-MA$_{16}$/PLLA single enantiomer solutions in HEPES buffered saline.

SEM experiments were performed on freeze-dried hydrogels using a LEO Gemini 1550 FEG-SEM, fitted with a field Emission Gun, and a voltage of 2 kV. Freeze-dried hydrogels were prepared by freezing in liquid nitrogen and subsequent freeze-drying at -50°C and $5\times10^{-7}$ bar overnight.

For the swelling/degradation tests, the hydrogel cylinders were placed in vials and after addition of 1 ml of HEPES buffered saline the hydrogels were allowed to swell at 37°C. The swelling experiment was performed in duplicate or triplicate. The swollen hydrogels were weighed at regular intervals after removal of the buffer. After each weighing the buffer was refreshed. The swelling ratio of the hydrogels was calculated from the initial hydrogel weight after hydrogel preparation ($W_0$) and the swollen hydrogel weight after exposure to buffer ($W_t$):

$$\text{Swelling ratio}: = \frac{W_t}{W_0}$$

### III.3 Synthesis 2

Two types of methacrylate functionalized poly(ethylene glycol)-poly(lactide) (PEG-PLA) star block copolymers, PEG-poly(L-lactide)-methacrylate (PEG-PLLA-MA) and PEG-poly(D-lactide)-methacrylate (PEG-PDLA-MA) (Figure 1A), and poly(ethylene glycol)-methacrylate/poly(L-lactide) (PEG-MA/PLLA) and poly(ethylene glycol)-methacrylate/poly (D-lactide) (PEG-MA/PDLA) (Figure 1B), were designed. PEG-PLLA-MA and PEG-PDLA-MA copolymers were prepared by a two-step synthesis procedure. First, eight-arm PEG-PLLA and PEG-PDLA star block copolymers with 12 lactyl units per PLA block (PEG-PLLA$_{12}$ and PEG-PDLA$_{12}$, $M_{n, PEG}$ = 21,800) were synthesized, as reported previously (Table 1, entry 1 and 2).[53] Subsequently, the PLA hydroxyl end groups were reacted with methacrylic anhydride using triethylamine (TEA) as a catalyst and dichloromethane (DCM) as a solvent at 30°C. The PEG-PLLA$_{12}$-MA and PEG-PDLA$_{12}$-MA copolymers were recovered by precipitation in a diethyl ether/hexane/methanol mixture (10/1/1 v/v) (Table 1, entry 3 and 4). $^1$H NMR showed a degree of methacrylation of ca. 40%, determined by comparing the integrals of the peaks corresponding to the methylene protons of the methacrylate group ($\delta$ 5.6 and 6.2) and the methylene protons of PEG ($\delta$ 3.6).

PEG-MA/PLA copolymers were prepared by a two-step synthesis procedure. First ca. 40% of the hydroxyl end groups of an eight-arm star PEG ($M_n$ = 21,800) were methacrylated. Subsequently, the ring opening polymerization of L-lactide or D-lactide was initiated by the remaining hydroxyl groups of methacrylate functionalized PEG, using the single-site Zn-complex as a catalyst and dichloromethane (DCM) as a solvent at room temperature. PEG-MA/PLLA and PEG-MA/PDLA copolymers were obtained by precipitation in a diethyl ether/methanol mixture (20/1 v/v). PEG-MA/PLA copolymers with 12 and 16 lactyl units per PLA block were prepared by varying the feeding ratio of lactide to PEG (Table 1, entry 5-8). The use of the single site Zn-catalyst allowed excellent control over the degree of polymerization of the PLA blocks and the methacrylation reaction was reproducible, giving similar degrees of methacrylation (Table 1).

**Table 1.** Synthesis of PEG-PLLA-MA and PEG-PDLA-MA, and PEG-MA/PLLA and PEG-MA/PDLA star block copolymers[a].

| Entry | Polymer | Lactide conversion (%) | $N_{LA}$[b] | | Degree of methacrylation (%) | $M_n \times 10^{-3}$ $^1$H NMR |
|---|---|---|---|---|---|---|
| | | | Theory[c] | $^1$H NMR | | |
| 1 | PEG-PLLA$_{12}$ | 94 | 12 | 12 | - | 28.7 |
| 2 | PEG-PDLA$_{12}$ | 96 | 12 | 12 | - | 28.7 |
| 3 | PEG-PLLA$_{12}$-MA | 94 | 12 | 12 | 40 | 28.8 |
| 4 | PEG-PDLA$_{12}$-MA | 96 | 12 | 12 | 42 | 28.9 |
| 5 | PEG-MA/PLLA$_{12}$ | 95 | 12 | 12 | 46 | 25.6 |
| 6 | PEG-MA/PDLA$_{12}$ | 94 | 12 | 12 | 46 | 25.6 |
| 7 | PEG-MA/PLLA$_{16}$ | 99 | 17 | 16 | 42 | 27.4 |

(continued)

| Entry | Polymer | Lactide conversion (%) | $N_{LA}$[b] Theory[c] | $^1$H NMR | Degree of methacrylation (%) | $M_n \times 10^{-3}$ $^1$H NMR |
|---|---|---|---|---|---|---|
| 8 | PEG-MA/ PDLA$_{16}$ | 95 | 16 | 16 | 42 | 27.4 |

[a] The ring opening polymerization of lactide was performed in DCM for 1 h at RT using PEG or partially methacrylate functionalized PEG as an initiator and the single site Zn-complex Zn(Et)[OC$_6$H$_3$(CH$_2$Me$_2$)-2-Me-4] as a catalyst, ([LA]$_0$ = 0.25 M, PEG hydroxyl groups : Zn catalyst = 2 : 1). The methacrylation was performed in DCM for 2 days at 30 °C ([OH]$_0 \approx$ 5 mM, MA : OH: TEA = 1 : 1.5: 1.1)
[b] Number of lactyl units per PLA block.
[c] Based on feed composition and conversion.

### III.4 Gelation by stereocomplexation.

The influence of the methacrylate groups and the PLA block length on stereocomplex hydrogel (denoted as stereo hydrogel) formation was studied at room temperature. Aqueous solutions of equimolar amounts of PEG-PLLA-MA and PEG-PDLA-MA, or PEG-MA/PLLA and PEG-MA/PDLA star block copolymers were mixed and after equilibration it was tested whether the sample had turned into a gel by the vial tilting method. Table 2 shows that the critical gel concentrations (CGCs) for stereocomplexation of PEG-PLA$_{12}$-MA and PEG-PLA$_{12}$ are equal, indicating that the methacrylate end groups do not influence the stereocomplexation. PEG-PLLA, PEG-PLLA-MA and PEG-MA/PLLA single enantiomers were also able to form gels at relatively high polymer concentrations. The CGC of PEG-PLLA$_{12}$-MA single enantiomer is somewhat lower compared to PEG-PLLA$_{12}$ single enantiomer, which is attributed to the increased hydrophobicity of PEG-PLLA$_{12}$-MA. Aqueous solutions of PEG-MA/PLLA$_{12}$ single enantiomer could be prepared up to much higher polymer concentrations compared to PEG-PLLA$_{12}$-MA single enantiomer. Stereo hydrogels could also be formed from PEG-MA/PLLA$_{12}$ and PEG-MA/PDLA$_{12}$ copolymers, but at much higher polymer concentrations compared to PEG-PLLA$_{12}$-MA and PEG-PDLA$_{12}$-MA copolymers. The higher CGC for stereocomplexation of PEG-MA/PLA$_{12}$ compared to PEG-PLA$_{12}$-MA is due to the lower crosslinking functionality (i.e. number of PLA blocks per molecule) and lower hydrophobicity of PEG-MA/PLA$_{12}$ compared to PEG-PLA$_{12}$-MA. Previously we have shown that the CGC for stereocomplexation of PLA-PEG-PLA triblock copolymers are higher compared to the CGC of eight-arm PEG-PLA star block copolymers.[13] PEG-MA/PLA$_{16}$ copolymers showed lower CGC values for stereocomplexation compared to PEG-MA/PLA$_{12}$ copolymers, due to the increased PLA block length.

Table 2. Critical gel concentrations (CGCs) of solutions containing PEG-PLLA, PEG-PLLA-MA and PEG-MA/PLLA single enantiomer star block copolymers or equimolar amounts of PEG-PLLA and PEG-PDLA, PEG-PLLA-MA and PEG-PDLA-MA, or PEG-MA/PLLA and PEG-MA/PDLA star block copolymers in deionized water at room temperature.

| Polymer | CGC single enantiomer (w/v%) | CGC mixed enantiomers (w/v%) |
|---|---|---|
| PEG-PLA$_{12}$ | 20 | 7.5 |
| PEG-PLA$_{12}$-MA | 17.5 | 7.5 |
| PEG-MA/PLA$_{12}$ | 30 | 22.5 |
| PEG-MA/PLA$_{16}$ | 20 | 12.5 |

### III.5 Rheology.

The mechanical properties of stereo hydrogels were studied by rheology experiments at 37°C. Stereo hydrogels were prepared by mixing aqueous solutions of equimolar amounts of PEG-PLLA$_{12}$ and PEG-PDLA$_{12}$, PEG-PLLA$_{12}$-MA and PEG-PDLA$_{12}$-MA, or PEG-MA/PLLA$_{12}$ and PEG-MAlPDLA$_{12}$ star block copolymers in HEPES buffered saline (pH 7) in a polymer concentration range of 12.5 to 17.5 w/v%. After mixing, the solutions were quickly applied to the rheometer and the evolutions of the storage modulus (G') and loss modulus (G") were recorded (Figure 2a). Due to fast gelation, the gelation point of PEG-PLA$_{12}$, PEG-PLA$_{12}$-MA and PEG-MA/PLA$_{16}$ in a polymer concentration range of 12.5 to 17.5 w/v% could not be determined by rheology. After application of the sample on the rheometer, ca. 1-2 min were needed to set the instrument before starting the measurement. This shows that stereo hydrogels of PEG-PLA$_{12}$, PEG-PLA$_{12}$-MA and PEG-MA/PLA$_{16}$ were formed within 1-2 min. The storage modulus increased in time due to the ongoing stereocomplexation, until reaching a plateau value, marking the end of the crosslinking process (Figure 2a). Figure 2a shows that the storage modulus evolutions and plateau values of PEG-PLA$_{12}$-MA and PEG-PLA$_{12}$ copolymers were similar, which agrees well with the vial tilting tests, indicating that the methacrylate groups hardly influence the stereocomplexation (Table 2). For PEG-PLA$_{12}$ and PEG-PLA$_{12}$-MA copolymers the storage modulus plateau value was reached within ca. 5 h after mixing (Figure 2a). In contrast, the storage moduli of PEG-MA/PLA$_{16}$ stereo hydrogels continuously increased over 48 h. The storage moduli of the stereo hydrogels increased from 2.4 to 12.5 kPa for PEG-PLA$_{12}$-MA and from 0.1 to 5.2 kPa for PEG-MA/PLA$_{16}$, upon increasing the polymer concentration from 12.5 to 15 w/v% (Figure 2b). The PEG-PLA$_{12}$-MA stereo hydrogels showed lower damping factors (tan $\delta$ = G''/G') compared to the PEG-MA/PLA$_{16}$ stereo hydrogels (Figure 2b), indicating a higher network perfection.

**III.6 In situ monitoring of mechanical properties during photopolymerization.**

The mechanical properties of photopolymerized hydrogels were determined by combined rheology and UV-irradiation (350-400 nm, 16 mW/cm$^2$) of PEG-PLA$_{12}$-MA or PEG-MA/PLA$_{16}$ stereo hydrogels (yielding stereo-photo hydrogels) or solutions of PEG-PLLA$_{12}$-MA or PEG-MA/PLLA$_{16}$ single enantiomers (yielding photo hydrogels) in HEPES buffered saline (pH 7) at 37 °C (Figure 3 and 4). Figure 3a shows that the gelation time of PEG-PLLA$_{12}$-MA single enantiomer decreased from ca. 3 to 0.5 min upon increasing the polymer concentration from 12.5 to 17.5 w/v% at 5 mol% initiator concentration (with respect to the methacrylate groups). The storage modulus plateau value was reached within ca. 8 min and increased from 0.9 to 4.1 kPa upon increasing the polymer concentration from 12.5 to 17.5 w/v% (Figure 3a). Figure 3b shows that the gelation time of PEG-PLLA$_{12}$-MA single enantiomer at 15 w/v% polymer concentration decreased rapidly with increasing initiator concentration. At initiator concentrations of 2 and 5 mol% (with respect to the methacrylate groups) the gelation times of PEG-PLLA$_{12}$-MA single enantiomer were 6.5 and 1.7 min, respectively. At 1 mol% initiator concentration the 15 w/v% PEG-PLLA$_{12}$-MA single enantiomer solution did not form a gel within 15 min (Figure 3b).

As shown earlier, a stereo hydrogel was formed within 1-2 min after mixing aqueous solutions of equimolar amounts of PEG-PLLA$_{12}$-MA and PEG-PDLA$_{12}$-MA copolymers. UV-irradiation of the stereo hydrogel at 1 mol% initiator and 15 w/v% polymer concentration 10 min after mixing increased the storage modulus from 5.6 to 9.6 kPa within 15 min due to photocrosslinking (Figure 4a). Here, an initiator concentration of 1 mol% (with respect to the methacrylate groups) corresponds to 0.003 wt%, which is very low compared to the commonly used concentration of 0.05 wt%.[49] Low initiator concentrations are preferred, due to toxicity of the initiator. The photocrosslinking at this low initiator concentration implies in turn that low light intensities may be used to obtain stereo-photo hydrogels.

The storage modulus of the stereo-photo hydrogel is highly dependent on the stereocomplex equilibration time before UV-irradiation. Figure 4b shows a plot of the ratio of the storage modulus of a PEG-PLA$_{12}$-MA stereo-photo hydrogel and the storage modulus plateau value of the corresponding stereo hydrogel (reached after ca. 5h, Figure 2a) as a function of the stereocomplex equilibration time. The storage modulus plateau value of the stereo-photo hydrogel (after 8 min of UV-irradiation) increased linearly with increasing the stereocomplex equilibration time at 15 w/v% polymer concentration and 5 mol% initiator concentration (corresponding to 0.015 wt%). This initiator concentration is low compared to the generally used concentration of 0.05 wt%.[49] UV-irradiation after 6 h of equilibration resulted in an almost 6-fold increase in the storage modulus of the PEG-PLA$_{12}$-MA stereo-photo hydrogel compared to the corresponding PEG-PLA$_{12}$-MA stereo hydrogel (31.6 vs. 5.6 kPa) and a 17-fold increase compared to the corresponding PEG-PLLA$_{12}$-MA photo hydrogel and (31.6 vs. 1.8 kPa). Since the hydrophobic methacrylate groups are at the PLA chain ends, the chemical crosslinks are most probably formed in the PLA domains. A schematic representation of the stereo and stereo-photo hydrogel preparation for PEG-PLA-MA and PEG-MA/PLA copolymers is shown in Figure 5. Furthermore, the photoinitiator used, Irgacure 2959, is rather hydrophobic (maximum concentration in water is 0.7 wt%[49]), and may therefore preferably partition into the hydrophobic PLA domains, thereby increasing the local initiator concentration and thus photopolymerization rate in these domains. Therefore, the increased storage modulus upon increased stereocomplex equilibration time may be due to the formation of more PLA domains, resulting in a more densely crosslinked network and increased photopolymerization conversion. PEG-MA/PLA$_{16}$ stereo-photo hydrogels also showed much higher storage moduli compared to the corresponding PEG-MA/PLLA$_{16}$ stereo or photo hydrogels (results not shown). Therefore, combining stereocomplexation and photocrosslinking may provide fast gelation *in vitro* and *in vivo*[55], yielding hydrogels with good mechanical properties.

**III.7 Morphology of Photopolymerized hydrogels.**

To study the influence of stereocomplexation on the morphology of photopolymerized hydrogels scanning electron microscopy (SEM) measurements were performed on freeze-dried PEG-PLA$_{12}$-MA and PEG-MA/PLA$_{16}$ stereo-photo and photo hydrogels. The stereo-photo and photo hydrogels were prepared by UVA-irradiation (250 mW/cm$^2$) of PEG-PLA$_{12}$-MA or PEG-MA/PLA$_{16}$ stereo hydrogels (equilibrated for ca. 15 min after mixing the enantiomeric solutions) and solutions of PEG-PLLA$_{12}$-MA or PEG-MA/PLLA$_{16}$ single enantiomers, respectively, in HEPES buffered saline (pH 7) at 8 mol% initiator and 15 w/v% polymer concentration. Figures 6A and 6B show that PEG-PLA$_{12}$-MA stereo-photo hydrogels have pore sizes of ca. 5 $\mu$m, while PEG-PLLA$_{12}$-MA photo hydrogels have pore sizes of ca. 10 $\mu$m, indicating that stereocomplexation has a significant influence on the pore size of the freeze-dried PEG-PLA-MA hydrogels. In contrast, freeze dried PEG-MA/PLA$_{16}$ stereo-photo hydrogels and PEG-MA/PLLA$_{16}$ photo hydrogels showed similar pore sizes (ca. 10 $\mu$m, Figure 6C and 6D). Apparently, the position of the crosslinking group has much influence on the pore size of freeze-dried stereo-photo hydrogels.

### III.8 Hydrogel swelling and degradation.

Hydrogels based on PEG-PLA-MA or PEG-MA/PLA copolymers were degradable under physiological conditions. To study the rate of degradation, stereo-photo and photo hydrogels were prepared by UVA-irradiation (250 mW/cm$^2$) of PEG-PLA$_{12}$-MA or PEG-MA/PLA$_{16}$ stereo hydrogels (equilibrated for ca. 15 min after mixing the enantiomeric solutions) and solutions containing PEG-PLLA$_{12}$-MA or PEG-MA/PLLA$_{16}$ single enantiomer, respectively, in HEPES buffered saline (pH 7) at 8 mol% initiator concentration. After the hydrogels were formed, HEPES buffered saline was applied on top and the gels were allowed to swell at 37°C. At regular time intervals, the swelling ratio was calculated by rationing the swollen hydrogel weight after exposure to buffer with the initial hydrogel weight after preparation (W$_t$/W$_0$). Figure 7a shows that the PEG-PLA$_{12}$-MA stereo-photo hydrogels swelled to ca. twice their initial weight within 1 day, independent of the polymer concentration. The swelling ratio of PEG-PLLA$_{12}$-MA photo hydrogels also doubled after 1 day at 15 w/v% polymer concentration (Figure 7a). After the initial swelling, the swelling ratio remained constant for the PEG-PLA$_{12}$-MA stereo-photo hydrogels, while the swelling ratio of PEG-PLLA$_{12}$-MA photo hydrogels continued to increase. In time, both hydrogels disintegrated, as shown by the decreasing swelling ratio, until they finally dissolved completely. The degradation time is defined as the time required to completely dissolve at least one of the two or three hydrogels used for testing one type of hydrogel. Figure 7a shows that the PEG-PLA$_{12}$-MA stereo-photo hydrogels were completely degraded after ca. 3 weeks and increasing the polymer concentration from 12.5 to 17.5 w/v% hardly affected the degradation time. Interestingly, the degradation time of the PEG-PLA$_{12}$-MA stereo hydrogels was twice as high as compared to the PEG-PLLA$_{12}$-MA photo hydrogels (ca. 3 vs. 1.5 weeks, Figure 7a). This may be due to a higher crosslinking density of PEG-PLA$_{12}$-MA stereo-photo hydrogels compared to PEG-PLLA$_{12}$-MA photo hydrogels, as was also shown by the rheology measurements. The PEG-MA/PLA$_{16}$ stereo-photo hydrogels swelled over a period of ca. 5 weeks until reaching ca. twice their initial weight, independent of the polymer concentration (Figure 7b). The ongoing swelling is most likely due to PLA degradation, upon which the physical crosslinks are lost, resulting in a less densely crosslinked network held together by only chemical crosslinks (Figure 8). PEG-MA/PLA$_{16}$ stereo-photo hydrogels with 12.5 w/v% polymer concentration completely degraded after 7 weeks, while at 15 and 17.5 w/v% polymer concentration the stereo-photo hydrogels retained their integrity after 16 weeks.

The much slower degradation of the PEG-MA/PLA$_{16}$ stereo-photo hydrogels compared to the PEG-PLA$_{12}$-MA stereo-photo hydrogels is attributed to the slower hydrolysis of ester bonds of the polymerized methacrylate groups compared to the ester bonds of the PLA blocks, which correlates well with the results obtained by Bryant et al. for photopolymerized PEG dimethacrylate and PEG-PLA dimethacrylate hydrogels. [56] PEG-PLA-MA stereo-photo hydrogels degrade mainly through hydrolysis of the ester bonds in the PLA block, upon which both physical and chemical crosslinks are lost (Figure 8). In contrast, PLA degradation in the PEG-MA/PLA stereo-photo hydrogels leads to the formation of a less densely, chemically crosslinked network with increased swelling (Figure 8). The swollen PEG-MA/PLA stereo-photo hydrogels finally degrade through hydrolysis of the ester bonds of the polymerized methacrylate groups. It is possible to combine PEG-PLA-MA and PEG-MA/PLA copolymers to vary the degradation time.

### IV. Conclusions

PEG-PLA-MA copolymers were prepared by methacrylation of ca. 40% of the PLA hydroxyl end groups of eight-arm PEG-PLA star block copolymers. PEG-MA/PLA copolymers were prepared by ring opening polymerization of lactide initiated by eight-arm star PEG with 40% of its hydroxyl end groups methacrylated. PEG-PLA-MA and PEG-MA/PLA stereocomplexed hydrogels could be rapidly formed in situ upon mixing aqueous solutions containing equimolar amounts of PEG-PLLA-MA and PEG-PDLA-MA, or PEG-MA/PLLA and PEG-MA/PDLA copolymers. Interestingly, stereocomplexation aided in the photopolymerization of the methacrylate groups. Photocrosslinking of stereo hydrogels, yielding stereo-photo hydrogels, resulted in increased hydrogel storage moduli, compared to the hydrogels crosslinked by only stereocomplexation (stereo hydrogels) or only photocrosslinking (photo hydrogels). Moreover, photocrosslinking of stereo hydrogels already took place at very low initiator concentrations. The degradation time of PEG-PLA-MA stereo-photo hydrogels was doubled compared to PEG-PLLA-MA photo hydrogels (ca. 3 vs. 1.5 weeks). PEG-MA/PLA stereo-photo hydrogels degraded within ca. 7 to over 16 weeks, depending on the polymer concentration. In principle, PEG-PLA-MA and PEG-MA/PLA may be combined to vary the hydrogel degradation rate. The fast gelation *in vitro* and *in vivo* due to stereocomplexation circumvents the need for fast photopolymerization, thus preventing substantial heat effects due to the photopolymerization and potentiating the use of low initiator concentrations and low light intensities. Moreover, the fast gelation allows for easy handling.

It will be understood that the stereo-photo hydrogels and in particular the process for their formation in situ within the human or animal body will have a high potential for in vivo applications including tissue engineering and drug delivery.

## V. References

1. Peppas, N. A.; Bures, P.; Leobandung, W.; Ichikawa, H. Eur. J. Pharm. and Biopharm. 2000, 50, 27-46.
2. Peppas, N. A.; Hilt, J. Z.; Khademhosseini, A.; Langer, R. Adv. Mater. 2006, 18, 1345-1360.
3. Qiu, Y.; Park, K. Adv. Drug Deliv. Rev. 2001, 53, 321-339.
4. Ruel-Gariepy, E.; Leroux, J. C. Eur. J. Pharm. Biopharm. 2004, 58, 409-426.
5. Nguyen, K. T.; West, J. L. Biomaterials 2002, 23, 4307-4314.
6. Temenoff, J. S.; Mikos, A. G. Biomaterials 2000, 21, 2405-2412.
7. Jeong, B.; Bae, Y. H.; Kim, S. W. Macromolecules 1999, 32, 7064-7069.
8. Zhong, Z. Y.; Dijkstra, P. J.; Feijen, J.; Kwon, Y. M.; Bae, Y. H.; Kim, S. W. Macromol. Chem. Phys. 2002, 203, 1797-1803.
9. Shim, W. S.; Kim, J. H.; Park, H.; Kim, K.; Kwon, I. C.; Lee, D. S. Biomaterials 2006, 27, 5178-5185.
10. Kang, G. D.; Cheon, S. H.; Khang, G.; Song, S. C. Eur. J. Pharm. Biopharm. 2006, 63, 340-346.
11. Seong, J. Y.; Jun, Y. J.; Jeong, B.; Sohn, Y. S. Polymer 2005, 46, 5075-5081.
12. de Jong, S. J.; van Eerdenbrugh, B.; van Nostrum, C. F.; Kettenes-van de Bosch, J. J.; Hennink, W. E. J. Contr. Rel. 2001, 71, 261-275.
13. Hiemstra, C.; Zhong, Z. Y.; Li, L. B.; Dijkstra, P. J.; Feijen, J. Biomacromolecules 2006, 7, 2790-2795.
14. Mukose, T.; Fujiwara, T.; Nakano, J.; Taniguchi, I.; Miyamoto, M.; Kimura, Y.; Teraoka, I.; Lee, C. W. Macromol. Biosci. 2004, 4, 361-367.
15. Li, S. M.; El Ghzaoui, A.; Dewinck, E. Macromol. Symp. 2005, 222, 23-35.
16. Li, J.; Ni, X. P.; Leong, K. W. J. Biomed. Mater. Res. 2003, 65A, 196-202.
17. Li, J.; Li, X.; Ni, X. P.; Wang, X.; Li, H. Z.; Leong, K. W. Biomaterials 2006, 27, 4132-4140.
18. Zhao, S. P.; Zhang, L. M.; Ma, D. J. Phys. Chem. B 2006, 110, 12225-12229.
19. Huh, K. M.; Cho, Y. W.; Chung, H.; Kwon, I. C.; Jeong, S. Y.; Ooya, T.; Lee, W. K.; Sasaki, S.; Yui, N. Macromol. Biosci. 2004, 4, 92-99.
20. Sabadini, E.; Cosgrove, T. Langmuir 2003, 19, 9680-9683.
21. Van Tomme, S. R.; van Steenbergen M. J.; De Smedt, S. C.; van Nostrum, C. F.; Hennink, W. E. Biomaterials 2005, 26, 2129-2135.
22. Ramachandran, S.; Tseng, Y.; Yu, Y. B. Biomacromolecules 2005, 6, 1316-1321.
23. Elbert, D. L.; Pratt, A. B.; Lutolf, M. P.; Halstenberg, S.; Hubbell, J. A.,J. Contr. Rel. 2001, 76, 11-25.
24. Lutolf, M. P.; Hubbell, J. A. Biomacromolecules 2003, 4, 713-722.
25. Lutolf, M. P.; Raeber, G. P.; Zisch, A. H.; Tirelli, N.; Hubbell, J. A. Adv. Mater. 2003, 15, 888-892.
26. Shu, X. Z.; Liu, Y. C.; Palumbo, F. S.; Lu, Y.; Prestwich, G. D. Biomaterials 2004, 25, 1339-1348.
27. Peattie, R. A.; Rieke, E. R.; Hewett, E. M.; Fisher, R. J.; Shu, X. Z.; Prestwich, G. D. Biomaterials 2006, 27, 1868-1875.
28. Ghosh, K.; Ren, X. D.; Shu, X. Z.; Prestwich, G. D.; Clark, R. A. F., Tissue Eng. 2006, 12, 601-613.
29. Hiemstra, C.; van der Aa, L. J.; Zhong, Z. Y.; Dijkstra, P. J.; Feijen, J., Macromolecules 2006, 2007,40,1165-1173.
30. Maia, J.; Ferreira, L.; Carvalho, R.; Ramos, M. A.; Gil, M..H. Polymer 2005, 46, 9604-9614.
31. Balakrishnan, B.; Jayakrishnan, A. Biomaterials 2005, 26, 3941-3951.
32. Yoshida, T.; Aoyagi, T.; Kokufuta, E.; Okano, T. J. Polym. Sci. Polym. Chem. 2003, 41, 779-787.
33. Cadee, J. A.; De Kerf, M.; De Groot, C. J.; Den Otter, W.; Hennink, W. E. Polymer 1999, 40, 6877-6881.
34. Kasper, F. K.; Seidlits, S. K.; Tang, A.; Crowther, R. S.; Carney, D. H.; Barry, M. A.; Mikos, A. G. J. Contr. Rel. 2005, 104, 521-539.
35. Oudshoorn, M. H. M.; Rissmann, R.; Bouwstra, J. A.; Hennink, W. E. Biomaterials 2006, 27, 5471-5479.
36. Temenoff, J. S.; Park, H.; Jabbari, E.; Conway, D. E.; Sheffield, T. L.; Ambrose, C. G.; Mikos, A. G. Biomacromolecules 2004, 5, 5-10.
37. Kim, S.; Chung, E. H.; Gilbert, M.; Healy, K. E. J. Biomed. Mater. Res. 2005, 75A, 73-88.
38. Baroli, B. J. Chem. Tech. And Biotechnol. 2006, 81, 491-499.
39. West, J. L.; Hubbell, J. A., Macromolecules 1999, 32, 241-244.
40. Bryant, S. J.; Anseth, K. S. J. Biomed. Mater. Res. 2002, 59, 63-72.

41. Bryant, S. J.; Bender, R. J.; Durand, K. L.; Anseth, K. S. Biotechnol. Bioeng. 2004, 86, 747-755.

42. Sawhney, A. S.; Pathak, C. P.; Hubbell, J. A., Macromolecules 1993, 26, 581-587.

43. Halstenberg, S.; Panitch, A.; Rizzi, S.; Hall, H.; Hubbell, J. A. Biomacromolecules 2002, 3, 710-723.

44. Park, Y. D.; Tirelli, N.; Hubbell, J. A., Biomaterials 2003, 24, 893-900.

45. Elisseeff, J.; Anseth, K.; Sims, D.; McIntosh, W.; Randolph, M.; Yaremchuk, M.; Langer, R. Plast. Reconstr. Surg. 1999, 104, (4), 1014-1022.

46. Yamaoka, T.; Tabata, Y.; Ikada, Y. J. Pharm. Sci. 1994, 83, 601-606.

47. Elisseeff, J.; Anseth, K.; Sims, D.; McIntosh, W.; Randolph, M.; Langer, R. Proc. Natl. Acad. Sci. Unit. States Am. 1999, 96, 3104-3107.

48. Burdick, J. A.; Peterson, A. J.; Anseth, K. S. Biomaterials 2001, 22, 1779-1786.

49. Bryant, S. J.; Nuttelman, C. R.; Anseth, K. S. J. Biomater. Sci. Polymer Edn. 2000, 11, 439-457.

50. Muggli, D. S.; Burkoth, A. K.; Keyser, S. A.; Lee, H. R.; Anseth, K. S., R. Macromolecules 1998, 31, 4120-4125.

51. Hiemstra, C.; Zhong, Z. Y.; Dijkstra, P. J.; Van Tomme, S. R.; Jacobs, J. J. L.; Den Otter, W.; Hennink, W. E.; Feijen, J. J. Contr. Rel. 2007, in press.

52. Bos, G. W.; Jacobs, J. J. L.; Koten, J. W.; Van Tomme, S. R.; Veldhuis, T. F. J.; van Nostrum, C. F.; Den Otter, W.; Hennink, W. E. Eur. J. Pharm. Sci. 2004, 21, 561-567.

53. Hiemstra, C.; Zhong, Z. Y.; Dijkstra, P. J.; Feijen, J. Macromol. Symp. 2005, 224, 119-131.

54. Lin-Gibson, S.; Bencherif, S.; Cooper, J. A.; Wetzel, S. J.; Antonucci, J. M.; Vogel, B. M.; Horkay, F.; Washburn, N. R. Biomacromolecules 2004, 5, 1280-1287.

55. Bos, G. W.; Hennink, W. E. Brouwer, L. A.; den Otter, W.; Veldhuis, T. F. J.; van Nostrum, C. F.; van Luyn, M. J. A. Biomaterials 2005, 26, 3901-3909.

56. Bryant, S. J.; Anseth, K. S. J. Biomed. Mater. Res. 2002, 64A, 70-79.

**Claims**

1. Stereo photo hydrogel formed by stereo complexed and photo cross-linked polymers, which polymers comprise two types of polymers having at least one hydrophilic component, at least one hydrophobic mutually stereo complexing component, and the two types of polymers comprise at least one mutually photo cross-linkable component.

2. Hydrogel according to claim 1, wherein the hydrophilic component comprises PEG, dextran, hyaluronic acid, pullulan, chondroitin sulfate, poly(vinyl alcohol), poly(hydroxyethyl methacrylate), poly(aspartic acid), poly(glutamic acid), poly(acrylic acid), an poly(($C_1$-$C_6$)alkyloxazoline), such as poly(methyl- or ethyl-oxazoline), and/or
wherein the hydopohobic stereo complexing component comprises poly (L-lactide) or poly (D-lactide), which poly (L- or D-lactide) preferably comprises 8-30, preferably 10-20, more preferably 12-16 lactyl units per poly (L- or D-lactide) and/or
wherein the photo cross-linkable component comprises acrylate, methacrylate, acrylamide and fumarate.

3. Hydrogel according to claim 1 or 2, wherein the photo cross-linkable component is cross-linkable using visible or (long wavelength) ultraviolet irradiation, and/or
wherein the photo cross-linking requires low intensity UV/irradiation, such as 0.05-20 mW/cm2, where there is a tissue barrier (such as intact skin), or from 2-20 mW/cm2 where there is no tissue barrier, or visible light at an intensity of the visible light of preferably from 30-100 mW/cm2.

4. Hydrogel according to any one of the claims 1-3, wherein the stereo complexing component and photo cross-linkable component are directly linked to the hydrophilic component or wherein the stereo complexing component links the photo cross-linkable component to the hydrophilic component.

5. Hydrogel according to any one of the claims 1-4, wherein the constituting polymers have the form of a triblok or of multi-arm structure, preferably a 3-12 arm structure, more preferably 8-10 arm structure.

6. Hydrogel according to any one of the claims 1-5, having a storage modules G'larger than 1 kPa, such as 1-100 kPa.

7. Process of making stereo photo hydrogel comprising the steps of

   a. providing a mixture of at least two types of polymers having at least one hydrophilic component, at least one hydrophobic mutually stereo complexing component and at least one of the types comprises at least one photo cross-linkable component;

b. stereo complexing the two types of polymers thereby forming a stereo complexed hydrogel; and

c. photo cross-linking the stereo complexed hydrogel using visible or UV irradiation, thereby forming the stereo photo hydrogel.

**8.** Process according to claim 7, wherein the two types of polymers comprise at least one mutually photo cross-linkable component, and/or

wherein the hydrophilic component comprises PEG, dextran, hyaluronic acid, pullulan, chondroitin sulfate, poly (vinyl, alcohol), poly(hydroxyethyl methacrylate), poly(aspartic acid), poly(glutamic acid) poly acrylic acid), poly(vinyl, alcohol), and poly((C1-C6)alkyloxazoline), such as poly(methyl- or ethyl-oxazoline) and/or

Wherein the hydrophobic stereo complexing component comprises poly (L-lactide) or poly (D-lactide), which poly (L- or D-lactide) preferably comprises 8-30, preferably 10-20, more preferably 12-16 lactyl units, and/or

Wherein the photo cross-linkable component comprses acrylate, methacrylate, acrylamide and fumarate and preferably the photo cross-linkable component is cross-linkable using visible or (long wavelength) ultraviolet radiation.

**9.** Process according to claim 8, wherein the photo cross-linking uses a cytocompatible photo initiatore, preferably in an amount of 0.001 to 0.05 wt%, such as 2.2-dimethoxy-2-phenylacetophenone (Irgacure 651), 1-hdyroxycyclhexyle phenyl ketone (Irgacure 184), 2-methyl-1-[4-(methylthio) phenyl]-2-(4-morpholinyl)-1-propanone (Irgacure 907), and 2-hydroxy-1-(hydroxyethoxy)phenyl]-2-methyl-1-propanone (Darocur 2959), camphorquinone (CQ) with ethyl 4-N, N-dimethylaminobenzoate (4EDMAB) and triethanolamine (TEA) and the photosensitizer isopropyl thioxanthone, and/or

Wherein the photo cross-linking requires low intensity UV-irragdiation, such as 0.05-20 mW/cm2 when there is a tissue barrier (such as intact skin), or from 2-20 mW/cm2 when there is no tissue barrier, or visible light at an intensity of preferably from 30-100 mW/cm2.

**10.** Process according to claim 8 or 9, wherein the stereo complexing component and photo cross-linkable component are directly linked to the hydrophilic component or

Wherein the stereo complexing component links the photo cross-linkable component to the hydrophilic component.

**11.** Process according to any one of the claims 8-10, wherein the constituting polymers have the form of a triblock or a multi-arm structure, preferably a 3-12 arm structure, more preferably 8-10 arm structure, and/or

wherein the polymer concentration is in the range of 5-30 w/v%, preferably 10-20 w/v%.

**12.** Process according to any one of the claims 8-11, wherein the time for the stereo complexing is in the rage of 1 min-3 days, preferably 2 hours-1 day, more preferably 4-10 hours.

**13.** Process according to any one of the claims 8-12, comprising the step of hydrolyzing the stereo complexing components thereby forming a swollen hydrogel.

**14.** Hydrogel obtainable with the process of any one of the claim 8-13, and preferably comprising a pharmaceutically active agent or moiety that binds a pharmaceutically active agent.

**15.** Polymers comprising two types of polymers having at least one hydrophilic component, at least one hydrophobic mutually stereo complexing component and at least one of the types comprises at least one photo cross-linkable component for use in making a hydrogel comprising a pharmaceutically active agent or moiety that binds a pharmaceutically active agent, within the human or animal body, which polymers are preferably stereo complexed.

**16.** Pharmaceutical kit for forming the hydrogel as defined in any one of claims 1 to 6, comprising two containers each comprising one of two types of polymers having at least one hydrophilic component, at least one hydrophobic mutually stereo complexing component, and at least one of the types comprises at least one photo cross-linkable component, and wherein the pharmaceutically active agent as defined in claim 14, is preferably contained in one or both container and/or in a separate container.

**Patentansprüche**

**1.** Stereophotohydrogel, das aus stereokomplexierten und photovernetzten Polymeren gebildet ist, wobei die Polymere zwei Arten von Polymeren mit mindestens einer hydrophilen Komponente und mindestens einer hydrophoben, wechselseitig stereokomplexierenden Komponente umfassen, und wobei die zwei Arten von Polymeren mindestens eine wechselseitig photovernetzbare Komponente umfassen.

2. Hydrogel gemäß Anspruch 1, wobei die hydrophile Komponente PEG, Dextran, Hyaluronsäure, Pullulan, Chondroitinsulfat, Poly(vinylalkohol), Poly(hydroxyethylmethacrylat), Poly(asparaginsäure), Poly(glutaminsäure), Poly(acrylsäure), ein Poly((C1-$C_6$)alkyloxazolin) wie etwa Poly(methyl- oder ethyloxazolin) umfasst, und/oder
wobei die hydrophobe, stereokomplexierende Komponente Poly(L-lactid) oder Poly(D-lactid) umfasst, wobei das Poly(L- oder D-lactid) bevorzugt 8-30, bevorzugt 10-20, mehr bevorzugt 12-16 Lactyleinheiten pro Poly(L- oder D-lactid) umfasst, und/oder
wobei die photovernetzbare Komponente Acrylat, Methacrylat, Acrylamid und Fumarat umfasst.

3. Hydrogel gemäß Anspruch 1 oder 2, wobei die photovernetzbare Komponente unter Verwendung von sichtbarer oder ultravioletter Strahlung (langer Wellenlänge) vernetzbar ist, und/oder
wobei die Photovernetzung UV-Strahlung geringer Intensität bedarf, wie etwa 0,05 bis 20 mW/cm$^2$, wo eine Gewebebarriere (wie etwa intakte Haut) besteht, oder von 2-20 mW/cm$^2$, wo keine Gewebebarriere besteht, oder sichtbares Licht bei einer Intensität von sichtbarem Licht von bevorzugt 30-100 mW/cm$^2$.

4. Hydrogel gemäß einem der Ansprüche 1 bis 3, wobei die stereokomplexierende Komponente und photovernetzbare Komponente direkt an die hydrophile Komponente gebunden sind, oder wobei die stereokomplexierende Komponente die photovernetzbare Komponente mit der hydrophilen Komponente verknüpft.

5. Hydrogel gemäß einem der Ansprüche 1-4, wobei die aufbauenden Polymere die Form eines Triblocks oder einer Multiarm-Struktur aufweisen, bevorzugt eine 3-12-Armstruktur, mehr bevorzugt 8-10-Armstruktur.

6. Hydrogel gemäß einem der Ansprüche 1-5, mit einem Speichermodul G' größer als 1 kPa, wie etwa 1-100 kPa.

7. Verfahren zur Herstellung von Stereophotohydrogel umfassend die Schritte

a. Bereitstellung einer Mischung von mindestens zwei Arten von Polymeren mit mindestens einer hydrophilen Komponente und mindestens einer hydrophoben wechselseitig stereokomplexierenden Komponente, und wobei mindestens eine der Arten mindestens eine photoverlinkbare Komponente umfasst;
b. Stereokomplexierung der zwei Arten von Polymeren, dabei ein stereokomplexiertes Hydrogel bildend; und
c. Photovernetzung des stereokomplexierten Hydrogels unter Verwendung von sichtbarer oder UV-Strahlung, dabei das Stereophotohydrogel bildend.

8. Verfahren gemäß Anspruch 7, wobei die zwei Arten von Polymeren mindestens eine wechselseitig vernetzbare Komponente umfassen, und/oder
wobei die hydrophile Komponente PEG, Dextran, Hyaluronsäure, Pullulan, Chondroitinsulfat, Poly(vinylalkohol), Poly(hydroxyethylmethacrylat), Poly(asparaginsäure), Poly(glutaminsäure), Poly(acrylsäure), Poly(vinyl, alkohol) und Poly(($C_1$-$C_6$)alkyloxazolin) wie etwa Poly(methyl- oder ethyloxazolin) umfasst, und/oder
wobei die hydrophobe, stereokomplexierende Komponente Poly(L-lactid) oder Poly(D-lactid) umfasst, wobei das Poly(L- oder D-lactid) bevorzugt 8-30, bevorzugt 10-20, mehr bevorzugt 12-16 Lactyleinheiten pro Poly(L- oder D-lactid) umfasst, und/oder
wobei die photovernetzbare Komponente Acrylat, Methacrylat, Acrylamid und Fumarat umfasst, und wobei bevorzugt die photovernetzbare Komponente unter Verwendung von sichtbarer oder ultravioletter Strahlung (langer Wellenlänge) vernetzbar ist.

9. Verfahren gemäß Anspruch 8, wobei die Photovernetzung einen cyto-kompatiblen Photoinitiator verwendet, bevorzugt in einer Menge von 0.001 bis 0.05 Gew.-%, wie etwa 2,2-Dimethoxy-2-phenylacetophenon (Irgacure 651), 1-Hydroxycyclohexylphenylketon (Irgacure 184), 2-Methyl-1-[4-(methylthio)phenyl]-2-(4-morpholinyl)-1-propanon (Irgacure 907) und 2-Hydroxy-1-(hydroxyethoxy)phenyl]-2-methyl-1-propanon (Darocur 2959), Campherchinon (CQ) mit Ethyl-4-N,N-dimethylaminobenzoat (4EDMAB) und Triethanolamin (TEA) und der Photosensibilisator Isopropylthioxanthon, und/oder wobei die Photovernetzung UV-Strahlung geringer Intensität bedarf, wie etwa 0,05-20 mW/cm$^2$, wo eine Gewebebarriere (wie etwa intakte Haut) besteht, oder von 2-20 mW/cm$^2$, wenn keine Gewebebarriere besteht, oder sichtbares Licht bei einer Intensität von bevorzugt 30-100 mW/cm$^2$.

10. Verfahren gemäß Anspruch 8 oder 9, wobei die stereokomplexierende Komponente und die photovernetzbare Komponente direkt an die hydrophile Komponente gebunden werden, oder
wobei die stereokomplexierende Komponente die photovernetzbare Komponente mit der hydrophilen Komponente verknüpft.

**11.** Verfahren gemäß einem der Ansprüche 8-10, wobei die aufbauenden Polymere die Form eines Triblocks oder einer Multiarm-Struktur aufweisen, bevorzugt eine 3-12 Armstruktur, mehr bevorzugt 8-10 Armstruktur, und/oder wobei die Polymerkonzentration im Bereich von 5-30 Massen-%, bevorzugt 10-20 Massen-% liegt.

**12.** Verfahren gemäß einem der Ansprüche 8-11, wobei die Zeit für die Stereokomplexierung im Bereich von 1 Minute - 3 Tagen, bevorzugt 2 Stunden - 1 Tag, mehr bevorzugt 4-10 Stunden liegt.

**13.** Verfahren gemäß einem der Ansprüche 8-12, das den Schritt der Hydrolysierung der stereokomplexierenden Komponenten umfasst, dabei ein gequollenes Hydrogel bildend.

**14.** Hydrogel, erhältlich durch ein Verfahren gemäß den Ansprüchen 8-13, und das bevorzugt einen pharmazeutisch aktiven Wirkstoff oder ein Strukturteil umfasst, das einen pharmazeutisch aktiven Wirkstoff bindet.

**15.** Polymere, die zwei Arten von Polymeren mit mindestens einer hydrophilen Komponente und mindestens einer hydrophoben, wechselseitig stereokomplexierenden Komponente umfassen, und wobei mindestens eine der (Polymer)Arten mindestens eine photovernetzbare Komponente umfasst, für die Verwendung bei der Herstellung eines Hydrogels, das einen pharmazeutisch aktiven Wirkstoff oder ein Strukturteil umfasst, das einen pharmazeutisch aktiven Wirkstoff bindet, im menschlichen oder tierischen Körper, wobei die Polymere bevorzugt stereokomplexiert sind.

**16.** Pharmazeutischer Kit zur Bildung eines Hydrogels wie in einem der Ansprüche 1 bis 6 definiert, der zwei Behälter umfasst, die jeweils eine von zwei Arten von Polymeren mit mindestens einer hydrophilen Komponente und mindestens einer hydrophoben, wechselseitig stereokomplexierenden Komponente umfasst, und wobei mindestens eine der (Polymer)Arten mindestens eine photoverlinkbare Komponente umfasst, und wobei die pharmazeutisch aktive Verbindung, wie in Anspruch 14 definiert, vorzugsweise in einem oder beiden Behältern und/oder in einem separaten Behälter enthalten ist.

**Revendications**

**1.** Stéréo-photo-hydrogel formé par des polymères stéréo-complexés et photo-réticulés, lesquels polymères comprennent deux types de polymères ayant au moins une composante hydrophile, au moins une composante hydrophobe mutuellement stéréo-complexante, et les deux types de polymères comprennent au moins une composante mutuellement photo-réticulable.

**2.** Hydrogel selon la revendication 1, dans lequel la composante hydrophile comprend du PEG, du dextran, de l'acide hyaluronique, du pullulan, de la chondroïtine sulfate, du poly(vinyl alcool), du poly(hydroxyéthyl méthacrylate), du poly(acide aspartique), du poly(acide glutamique), du poly(acide acrylique), une poly($(C_1$-$C_6)$alkyloxazoline), telle qu'une poly(méthyl- ou éthyl-oxazoline), et/ou
dans lequel la composante hydrophobe stéréo-complexante comprend un poly (L-lactide) ou poly (D-lactide), lequel poly (L- ou D-lactide) comprend de préférence 8 à 30, de préférence 10 à 20, de manière davantage préférée 12 à 16 unités lactyle pour chaque poly (L- ou D-lactide) et/ou
dans lequel le composant photo-réticulable comprend de l'acrylate, du méthacrylate, de l'acrylamide et du fumarate.

**3.** Hydrogel selon la revendication 1 ou 2, dans lequel la composante photo-réticulable est réticulable en utilisant un rayonnement visible ou ultraviolet (lointain), et/ou
dans lequel la photoréticulation nécessite un rayonnement UV de faible intensité, telle que 0,05 à 20 $mW/cm^2$, quand il existe une barrière tissulaire (telle qu'une peau intacte), ou de 2 à 20 $mW/cm^2$ quand il n'y a pas de barrière tissulaire, ou de la lumière visible à une intensité de lumière visible de préférence de 30 à 100 $mW/cm^2$.

**4.** Hydrogel selon l'une quelconque des revendications 1 à 3, dans lequel la composante stéréo-complexante et la composante photo-réticulable sont liées directement à la composante hydrophile ou dans lequel la composante stéréo-complexante relie la composante photo-réticulable à la composante hydrophile.

**5.** Hydrogel selon l'une quelconque des revendications 1 à 4, dans lequel les polymères constitutifs ont la forme d'un tribloc ou d'une structure à bras multiples, de préférence une structure à 3 à 12 bras, et de manière davantage préférée une structure à 8 à 10 bras.

**6.** Hydrogel selon l'une quelconque des revendications 1 à 5, ayant un module de conservation G' supérieur à 1 kPa, tel que 1 à 100 kPa.

**7.** Procédé de fabrication d'un stéréo-photo-hydrogel comprenant les étapes consistant à

a. fournir un mélange d'au moins deux types de polymères ayant au moins une composante hydrophile, au moins une composante hydrophobe mutuellement stéréo-complexante et au moins l'un des types comprenant au moins une composante photo-réticulable ;
b. stéréo-complexer les deux types de polymères en formant ainsi un hydrogel stéréo-complexé ; et
c. photo-réticuler l'hydrogel stéréo-complexé en utilisant un rayonnement visible ou UV, en formant ainsi le stéréo-photo hydrogel.

**8.** Procédé selon la revendication 7, dans lequel les deux types de polymères comprennent au moins une composante mutuellement photo-réticulable, et/ou

dans lequel la composante hydrophile comprend du PEG, du dextran, de l'acide hyaluronique, du pullulan, de la chondroïtine sulfate, du poly(vinyl alcool), du poly(hydroxyéthyl méthacrylate), du poly(acide aspartique), du poly(acide glutamique), du poly(acide acrylique), une poly(($C_1$-$C_6$)alkyloxazoline), telle qu'une poly(méthyl- ou éthyl-oxazoline), et/ou

dans lequel la composante hydrophobe stéréo-complexante comprend un poly (L-lactide) ou poly (D-lactide), lequel poly (L- ou D-lactide) comprend de préférence 8 à 30, de préférence 10 à 20, de manière davantage préférée 12 à 16 unités lactyle, et/ou

dans lequel la composante photo-réticulable comprend de l'acrylate, du méthacrylate, de l'acrylamide et du fumarate et

de préférence la composante photo-réticulable est réticulable en utilisant un rayonnement visible ou ultraviolet (lointain).

**9.** Procédé selon la revendication 8, dans lequel la photoréticulation utilise un photo-initiateur cytocompatible, de préférence dans une quantité de 0,001 % à 0,05 % en poids, tel que la 2,2-diméthoxy-2-phénylacétophénone (Irgacure 651), la 1-hdyroxycyclhexyle phényl cétone (Irgacure 184), la 2-méthyl-1-[4-(méthylthio) phényl]-2-(4-morpholinyl)-1-propanone (Irgacure 907), et la 2-hydroxy-1-(hydroxyéthoxy)phényl]-2-méthyl-1-propanone (Darocur 2959), la camphorquinone (CQ) avec l'éthyl 4-N,N-diméthylaminobenzoate (4EDMAB) et la triéthanolamine (TEA) et le photosensibilisateur isopropyl thioxanthone, et/ou

dans lequel la photoréticulation nécessite un rayonnement UV de faible intensité, telle que de 0,05 à 20 mW/cm$^2$ quand il existe une barrière tissulaire (telle que de la peau intacte), ou de 2 à 20 mW/cm$^2$ quand il n'y a pas de barrière tissulaire, ou de la lumière visible à une intensité de préférence de 30 à 100 mW/cm$^2$.

**10.** Procédé selon la revendication 8 ou 9, dans lequel la composante stéréo-complexante et la composante photo-réticulable sont liées directement à la composante hydrophile ou

dans lequel la composante stéréo-complexante relie la composante photo-réticulable à la composante hydrophile.

**11.** Procédé selon l'une quelconque des revendications 8 à 10, dans lequel les polymères constitutifs ont la forme d'un tribloc ou d'une structure à bras multiples, de préférence une structure à 3 à 12 bras, de manière préférée une structure à 8 à 10 bras, et/ou

dans lequel la concentration de polymères est dans le domaine de 5 à 30 % poids/v, de préférence 10 à 20 % poids/v.

**12.** Procédé selon l'une quelconque des revendications 8 à 11, dans lequel la durée pour la stéréo-complexation est comprise dans le domaine de 1 min à 3 jours, de préférence 2 heures à 1 jour, de manière davantage préférée 4 à 10 heures.

**13.** Procédé selon l'une quelconque des revendications 8 à 12, comprenant l'étape d'hydrolyse des composantes stéréo-complexantes en formant ainsi un hydrogel gonflé.

**14.** Hydrogel susceptible d'être obtenu avec le procédé selon l'une quelconque des revendications 8 à 13, et comprenant de préférence un agent pharmaceutiquement actif ou un groupe qui se lie à un agent pharmaceutiquement actif.

**15.** Polymères comprenant deux types de polymères ayant au moins une composante hydrophile, au moins une composante hydrophobe mutuellement stéréo-complexante et au moins l'un des types comprenant au moins une composante photo-réticulable pour leurs utilisations pour la préparation d'un hydrogel comprenant un agent ou radical pharmaceutiquement actif ou un groupe qui se lie à un agent pharmaceutiquement actif, dans le corps humain ou animal, lesquels polymères sont de préférence stéréo-complexés.

**16.** Kit pharmaceutique pour former l'hydrogel tel que défini selon l'une quelconque des revendications 1 à 6, comprenant deux conteneurs comprenant chacun un des deux types de polymères ayant au moins une composante hydrophile, au moins une composante hydrophobe mutuellement stéréo-complexant, et au moins l'un des types comprenant au moins une composante photoréticulable, et où l'agent pharmaceutiquement actif tel que défini dans la revendication 14, est contenu de préférence dans un ou les deux conteneurs et/ou dans un conteneur séparé.

EP 2 147 031 B1

PLA   PEG

Eight-arm PEG-PLA-MA star block copolymer

## FIG. 1A

EP 2 147 031 B1

PLA

PEG

Eight-arm PEG-MA/PLA star block copolymer

## FIG. 1B

FIG. 2A

FIG. 2B

FIG. 3A

FIG. 3B

FIG. 4A

FIG. 4B

FIG. 5

FIG. 6

# PEG-PLA$_{12}$-MA

- ■ 12.5 w/v% D+L
- □ 15 w/v% D+L
- ▲ 17.5 w/v% D+L
- △ 15 w/v% L

FIG. 7A

# PEG-MA/PLA$_{16}$ D+L

FIG. 7B

PLA degradation
~3 weeks

**PEG-PLA-MA
stereo-photo hydrogel**

**clear solution**

PLA
degradation

degradation of ester
bonds of MA groups
7 - <21 weeks

**PEG-MA/PLA
stereo-photo hydrogel**

**swollen hydrogel**

**clear solution**

| PEG ⌐— | PLLA 〜〜 |
|---|---|
| MA ● | PDLA 〜〜 |

FIG. 8

EP 2 147 031 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030087985 A

**Non-patent literature cited in the description**

- **Peppas, N. A. ; Bures, P. ; Leobandung, W. ; Ichikawa, H.** *Eur. J. Pharm. and Biopharm.,* 2000, vol. 50, 27-46
- **Peppas, N. A. ; Hilt, J. Z. ; Khademhosseini, A. ; Langer, R.** *Adv. Mater,* 2006, vol. 18, 1345-1360
- **Qiu, Y. ; Park, K.** *Adv. Drug Deliv. Rev.,* 2001, vol. 53, 321-339
- **Ruel-Gariepy, E. ; Leroux, J. C.** *Eur. J. Pharm. Biopharm.,* 2004, vol. 58, 409-426
- **Nguyen, K. T. ; West, J. L.** *Biomaterials,* 2002, vol. 23, 4307-4314
- **Temenoff, J. S. ; Mikos, A. G.** *Biomaterials,* 2000, vol. 21, 2405-2412
- **Jeong, B. ; Bae, Y. H. ; Kim, S. W.** *Macromolecules,* 1999, vol. 32, 7064-7069
- **Zhong, Z. Y. ; Dijkstra, P. J. ; Feijen, J. ; Kwon, Y. M. ; Bae, Y. H. ; Kim, S. W.** *Macromol. Chem. Phys.,* 2002, vol. 203, 1797-1803
- **Shim, W. S. ; Kim, J. H. ; Park, H. ; Kim, K. ; Kwon, I. C. ; Lee, D. S.** *Biomaterials,* 2006, vol. 27, 5178-5185
- **Kang, G. D. ; Cheon, S. H. ; Khang, G. ; Song, S. C.** *Eur. J. Pharm. Biopharm.,* 2006, vol. 63, 340-346
- **Seong, J. Y. ; Jun, Y. J. ; Jeong, B. ; Sohn, Y. S.** *Polymer,* 2005, vol. 46, 5075-5081
- **de Jong, S. J. ; van Eerdenbrugh, B. ; van Nostrum, C. F. ; Kettenes-van de Bosch, J. J. ; Hennink, W. E.** *J. Contr. Rel.,* 2001, vol. 71, 261-275
- **Hiemstra, C. ; Zhong, Z. Y. ; Li, L. B. ; Dijkstra, P. J. ; Feijen, J.** *Biomacromolecules,* 2006, vol. 7, 2790-2795
- **Mukose, T. ; Fujiwara, T. ; Nakano, J. ; Taniguchi, I. ; Miyamoto, M. ; Kimura, Y. ; Teraoka, I. ; Lee, C. W.** *Macromol. Biosci.,* 2004, vol. 4, 361-367
- **Li, S. M. ; El Ghzaoui, A. ; Dewinck, E.** *Macromol. Symp.,* 2005, vol. 222, 23-35
- **Li, J. ; Ni, X. P. ; Leong, K. W.** *J. Biomed. Mater. Res.,* 2003, vol. 65A, 196-202
- **Li, J. ; Li, X. ; Ni, X. P. ; Wang, X. ; Li, H. Z. ; Leong, K. W.** *Biomaterials,* 2006, vol. 27, 4132-4140
- **Zhao, S. P. ; Zhang, L. M. ; Ma, D.** *J. Phys. Chem. B,* 2006, vol. 110, 12225-12229
- **Huh, K. M. ; Cho, Y. W. ; Chung, H. ; Kwon, I. C. ; Jeong, S. Y. ; Ooya, T. ; Lee, W. K. ; Sasaki, S. ; Yui, N.** *Macromol. Biosci.,* 2004, vol. 4, 92-99
- **Sabadini, E. ; Cosgrove, T.** *Langmuir,* 2003, vol. 19, 9680-9683
- **Van Tomme, S. R. ; van Steenbergen M. J. ; De Smedt, S. C. ; van Nostrum, C. F. ; Hennink, W. E.** *Biomaterials,* 2005, vol. 26, 2129-2135
- **Ramachandran, S. ; Tseng, Y. ; Yu, Y. B.** *Biomacromolecules,* 2005, vol. 6, 1316-1321
- **Elbert, D. L. ; Pratt, A. B. ; Lutolf, M. P. ; Halstenberg, S. ; Hubbell, J. A.** *J. Contr. Rel.,* 2001, vol. 76, 11-25
- **Lutolf, M. P. ; Hubbell, J. A.** *Biomacromolecules,* 2003, vol. 4, 713-722
- **Lutolf, M. P. ; Raeber, G. P. ; Zisch, A. H. ; Tirelli, N. ; Hubbell, J. A.** *Adv. Mater.,* 2003, vol. 15, 888-892
- **Shu, X. Z. ; Liu, Y. C. ; Palumbo, F. S. ; Lu, Y. ; Prestwich, G. D.** *Biomaterials,* 2004, vol. 25, 1339-1348
- **Peattie, R. A. ; Rieke, E. R. ; Hewett, E. M. ; Fisher, R. J. ; Shu, X. Z. ; Prestwich, G. D.** *Biomaterials,* 2006, vol. 27, 1868-1875
- **Ghosh, K. ; Ren, X. D. ; Shu, X. Z. ; Prestwich, G. D. ; Clark, R. A. F.** *Tissue Eng.,* 2006, vol. 12, 601-613
- **Hiemstra, C. ; van der Aa, L. J. ; Zhong, Z. Y. ; Dijkstra, P. J. ; Feijen, J.** *Macromolecules,* 2006, vol. 2007 (40), 1165-1173
- **Maia, J. ; Ferreira, L. ; Carvalho, R. ; Ramos, M. A. ; Gil, M..H.** *Polymer,* 2005, vol. 46, 9604-9614
- **Balakrishnan, B. ; Jayakrishnan, A.** *Biomaterials,* 2005, vol. 26, 3941-3951
- **Yoshida, T. ; Aoyagi, T. ; Kokufuta, E. ; Okano, T.** *J. Polym. Sci. Polym. Chem.,* 2003, vol. 41, 779-787
- **Cadee, J. A. ; De Kerf, M. ; De Groot, C. J. ; Den Otter, W. ; Hennink, W. E.** *Polymer,* 1999, vol. 40, 6877-6881

- **Kasper, F. K. ; Seidlits, S. K. ; Tang, A. ; Crowther, R. S. ; Carney, D. H. ; Barry, M. A. ; Mikos, A. G.** *J. Contr. Rel.,* 2005, vol. 104, 521-539
- **Oudshoorn, M. H. M. ; Rissmann, R. ; Bouwstra, J. A. ; Hennink, W. E.** *Biomaterials,* 2006, vol. 27, 5471-5479
- **Temenoff, J. S. ; Park, H. ; Jabbari, E. ; Conway, D. E. ; Sheffield, T. L. ; Ambrose, C. G. ; Mikos, A. G.** *Biomacromolecules,* 2004, vol. 5, 5-10
- **Kim, S. ; Chung, E. H. ; Gilbert, M. ; Healy, K. E.** *J. Biomed. Mater. Res.,* 2005, vol. 75A, 73-88
- **Baroli, B.** *J. Chem. Tech. And Biotechnol.,* 2006, vol. 81, 491-499
- **West, J. L. ; Hubbell, J. A.** *Macromolecules,* 1999, vol. 32, 241-244
- **Bryant, S. J. ; Anseth, K. S.** *J. Biomed. Mater. Res.,* 2002, vol. 59, 63-72
- **Bryant, S. J. ; Bender, R. J. ; Durand, K. L. ; Anseth, K. S.** *Biotechnol. Bioeng.,* 2004, vol. 86, 747-755
- **Sawhney, A. S. ; Pathak, C. P. ; Hubbell, J. A.** *Macromolecules,* 1993, vol. 26, 581-587
- **Halstenberg, S. ; Panitch, A. ; Rizzi, S. ; Hall, H. ; Hubbell, J. A.** *Biomacromolecules,* 2002, vol. 3, 710-723
- **Park, Y. D. ; Tirelli, N. ; Hubbell, J. A.** *Biomaterials,* 2003, vol. 24, 893-900
- **Elisseeff, J. ; Anseth, K. ; Sims, D. ; McIntosh, W. ; Randolph, M. ; Yaremchuk, M. ; Langer, R.** *Plast. Reconstr. Surg.,* 1999, vol. 104 (4), 1014-1022
- **Yamaoka, T. ; Tabata, Y. ; Ikada, Y.** *J. Pharm. Sci.,* 1994, vol. 83, 601-606
- **Elisseeff, J. ; Anseth, K. ; Sims, D. ; McIntosh, W. ; Randolph, M. ; Langer, R.** *Proc. Natl. Acad. Sci. Unit. States Am.,* 1999, vol. 96, 3104-3107
- **Burdick, J. A. ; Peterson, A. J. ; Anseth, K. S.** *Biomaterials,* 2001, vol. 22, 1779-1786
- **Bryant, S. J. ; Nuttelman, C. R. ; Anseth, K. S.** *J. Biomater. Sci. Polymer Edn.,* 2000, vol. 11, 439-457
- **Muggli, D. S. ; Burkoth, A. K. ; Keyser, S. A. ; Lee, H. R. ; Anseth, K. S., R.** *Macromolecules,* 1998, vol. 31, 4120-4125
- **Hiemstra, C. ; Zhong, Z. Y. ; Dijkstra, P. J. ; Van Tomme, S. R. ; Jacobs, J. J. L. ; Den Otter, W. ; Hennink, W. E. ; Feijen, J.** *J. Contr. Rel.,* 2007
- **Bos, G. W. ; Jacobs, J. J. L. ; Koten, J. W. ; Van Tomme, S. R. ; Veldhuis, T. F. J. ; van Nostrum, C. F. ; Den Otter, W. ; Hennink, W. E.** *Eur. J. Pharm. Sci.,* 2004, vol. 21, 561-567
- **Hiemstra, C. ; Zhong, Z. Y. ; Dijkstra, P. J. ; Feijen, J.** *Macromol. Symp.,* 2005, vol. 224, 119-131
- **Lin-Gibson, S. ; Bencherif, S. ; Cooper, J. A. ; Wetzel, S. J. ; Antonucci, J. M. ; Vogel, B. M. ; Horkay, F. ; Washburn, N. R.** *Biomacromolecules,* 2004, vol. 5, 1280-1287
- **Bos, G. W. ; Hennink, W. E. ; Brouwer, L. A. ; den Otter, W. ; Veldhuis, T. F. J. ; van Nostrum, C. F. ; van Luyn, M. J. A.** *Biomaterials,* 2005, vol. 26, 3901-3909
- **Bryant, S. J. ; Anseth, K. S.** *J. Biomed. Mater. Res.,* 2002, vol. 64A, 70-79